# EUROPEAN PATENT SPECIFICATION

(11) **EP 0 539 490 B1**
(45) Date of publication and mention of the grant of the patent: **08.11.1995**
(21) Application number: 91913696.0
(22) Date of filing: 12.07.1991
(51) Int. Cl.: C12Q 1/68, C12N 15/81, C12Q 1/02

(54) **LIBRARY SCREENING METHOD**
VERFAHREN ZUM ABSUCHEN EINER BIBLIOTHEK
PROCEDE DE TRIAGE DE BANQUES

(30) Priority: 13.07.1990 US 552183
(43) Date of publication of application: 05.05.1993
(73) Proprietor: TRANSKARYOTIC THERAPIES, INC., Cambridge, MA 02139 (US)
(72) Inventor: TRECO, Douglas, A., Arlington, MA 02174 (US); MILLER, Allan, M., Medford, MA 02155 (US)
(74) Representative: Holdcroft, James Gerald, Dr.
(86) International application number: PCT/US91/04926
(87) International publication number: WO 92/01069

(56) References cited:
- METHODS IN ENZYMOLOGY, vol. 101, 1983, New York, NY (US); R.J. ROTHSTEIN, pp. 202-210/
- NATURE, vol. 305, 15 September 1983, London (GB); A.W. MURRAY et al., pp. 189-193/

## Description

### Background of the Invention

A genomic DNA "library" is formed by digesting genomic DNA from a particular organism with a suitable restriction enzyme, joining the genomic DNA fragments to vectors and introducing the DNA fragment-containing vectors into a population of host cells. Complementary DNA (cDNA) is DNA which has been produced by an enzyme known as reverse transcriptase which can synthesize a complementary strand of DNA (cDNA) using a mRNA strand as a template. A cDNA library is formed by digesting cDNA with a suitable restriction enzyme, joining the cDNA fragments to vectors and introducing the cDNA fragment-containing vectors into a population of host cells. For example, Molecular Cloning by Sambrook, Fritsch & Maniatis, 2nd Ed., pages 9.5-9.6 describes the setting up of a DNA fragment library by introducing the DNA fragments as yeast artificial chromosomes (YAC's) in yeast host cells.

In producing a DNA or cDNA library, the pieces of DNA have been fragmented into an unordered collection of thousands or millions of pieces. To isolate a host cell carrying a specific DNA sequence (i.e., a specific DNA clone), the entire library must be screened. Radioactively labeled or otherwise labeled nucleic acid probes are traditionally employed to screen a DNA or cDNA library. Nucleic acid probes identify a specific DNA sequence by a process of in vitro hybridization between complementary DNA sequences in the probe and the DNA clone.

A specific DNA clone that has been identified and isolated in this manner can contain DNA that is contiguous to the probe sequence. A terminus of the DNA clone, therefore, can be used as a new probe to rescreen the same or another DNA library to obtain a second DNA clone which has an overlapping sequence with the first DNA clone. By obtaining a set of overlapping DNA clones, a physical map of a genomic region on a chromosome may be constructed. This process is called "chromosome walking" because each overlapping DNA clone which is isolated is one step further along the chromosome. Each DNA clone also can be studied to determine its physical relationship to a previously mapped genetic function and, thus, a series of overlapping DNA clones provides a physical map of a chromosome which is correlated to a map of genetic functions.

Chromosome walking is used, for example, to identify or localize a gene of interest, such as one thought to be causative of a disease or other condition. This is done by using a DNA fragment which displays a restriction fragment length polymorphism (RFLP) shown to be genetically linked to (i.e., physically localized to the same chromosome region as) a gene which causes or is associated with a disease, condition or phenotype, or a segment of DNA contiguous to such a RFLP, as an in vitro hybridization probe to screen a DNA library and pull out larger fragments of DNA in which all or part of the probe sequence is represented.

The usefulness of any DNA clone isolated in this manner is that it includes DNA that is contiguous to the RFLP sequence that is incrementally closer to the position of the sought-after gene than the original RFLP. To get a step closer, a labeled molecule corresponding to an end of the newly isolated DNA clone is prepared and used to rescreen the library, with the goal being to isolate DNA clones that overlap with sequences found in the first DNA clone and that are incrementally closer to the gene of interest than either the starting probe or the first DNA clone isolated. This procedure is repeated as needed, with the resulting DNA clones being used in genetic studies to assess whether they are more closely linked to the gene of interest. To walk over a distance of 10 million base pairs using presently-available chromosome walking techniques could require from 100 to 2,000 steps, depending on the DNA cloning vector system used. Any approach designed to decrease the work required to take a single walking step, or which would allow multiple walking projects to be carried out simultaneously would be a major advance.

The number of DNA clones which would be required to form a complete library of genomic DNA is determined by the size of the genome and the DNA clone capacity of the vector used to clone and propagate the segments of the genomic DNA. Construction and screening of genomic DNA libraries of organisms with large genomes is labor intensive and time consuming. The development of vectors having a capacity for large DNA clones has helped to reduce the labor involved in screening genomic libraries. However, screening libraries remains time consuming, slow and labor intensive.

### Summary of the Invention

The present invention is a method of identifying and isolating a DNA fragment of interest (a target DNA fragment), from a DNA fragment library in a eukaryotic host cell, which is based on homologous recombination between the target DNA fragment and DNA present in a targeting vector introduced into the DNA fragment library. It further relates to targeting vectors and DNA fragment libraries constructed in eukaryotic host cells as described herein.

The method of the present invention is used to screen a DNA fragment library constructed in a eukaryotic host cell in which genetic recombination (exchange of information between DNA present in a chromosome in the host cell and DNA introduced into the host cell) occurs by means of homologous recombination. In one embodiment, in which the eukaryotic host cell is yeast, genetic recombination occurs essentially exclusively by homologous recombination. DNA fragments in host cells are propagated in the form of artificial chromosomes which include, in addition to a DNA fragment insert, all of the DNA sequences necessary for the chromosome to participate in host cell replication and mitotic segregation in the same manner as naturally-present host cell chromosomes. In general, the artificial chromosome is present in one copy or low-copy number in a host cell.

The present method makes use of a targeting vector or vehicle which: 1) includes a DNA sequence, referred to as targeting DNA, homologous to at least a portion of the target DNA fragment and a selectable marker gene which is functional in host cells under appropriate conditions and 2) is non-replicating in the host cell. Preferably a double-strand break is made in the targeting DNA present in the targeting vector, which generally is circular when purified from an E. coli host. Alternatively, a gap can be introduced by making two cuts in the targeting DNA (e.g., with appropriately selected restriction enzyme(s)). The break or gap renders the vector linear, provides DNA ends which stimulate homologous recombination with host cell artificial chromosome sequences and increases the efficiency of stable transformation by homologous recombination.

The targeting vector is introduced into cells harboring the DNA fragment library, producing a mixed population of host cells, some of which contain the targeting vector and some of which do not. The resulting population of host cells is maintained under conditions appropriate for homologous recombination between DNA already present in the cell (i.e., prior to introduction of the targeting vector) and homologous sequences, such as those in the targeting vector. Subsequently, the population of cells is subjected to conditions appropriate for selection of host cells in which homologous recombination has occurred. Because the targeting vector is unable to replicate in the host cell, stable transformation with the selectable marker gene can occur only through homologous recombination. The selectable marker gene is replicated and, therefore, confers a stable phenotype, only in host cells in which homologous recombination has occurred. Identification of such host cells--and, thus, of host cells containing the DNA fragment of interest--is carried out by culturing the population of host cells under conditions (e.g., culturing on appropriate media) in which only those host cells in which homologous recombination and stable transformation with sequences which are replicatableoccurred can survive. Growth of a transformed host cell is indicative of the presence of the target DNA fragment. Host cells containing a target DNA fragment are, as a result, separated or isolated from host cells which do not contain the target DNA fragment. The target DNA fragment can be removed from the host cell and sequenced or manipulated (e.g., subcloned or mapped), using known techniques.

Alternatively, targeting DNA and a selectable marker gene for selection in yeast can be introduced into yeast host cells containing the DNA fragment library by mating a yeast strain containing the targeting DNA and the selectable marker gene on a replicating yeast linear plasmid with the yeast host cells containing the library. In this embodiment, the two yeast strains must be of opposite mating types. Homologous recombination occurs between the targeting linear plasmid and a library YAC having DNA homologous to targeting DNA, producing two linear molecules, each of which is a YAC. In one embodiment, the linear plasmid has negatively selectable markers flanking the targeting DNA sequence. Each of the two recombination products carries one of the two negatively selectable markers, making differential selection of the two recombination products possible. In another embodiment of the method in which mating of opposite yeast strain types is used, a yeast replicating plasmid is constructed in such a manner that the targeting DNA and a first selectable marker gene can be freed from the yeast replicon by recombination events and a second selectable marker gene, which is a negatively selectable marker gene, is used to select the replicon itself.

The replicating yeast plasmids described above can be introduced into host cells containing YACs by transformation.

In a preferred embodiment, the DNA fragment library is constructed in yeast, such as Saccharomyces (S.) cerevisiae or Schizosaccharomyces (S.) pombe, in which DNA fragments are present in yeast artificial chromosomes (YAC). Each yeast host cell contains one YAC or a few YACs, each present in one or few copies. A YAC includes, in addition to a DNA fragment, all of the DNA sequences required for chromosomes to replicate in yeast, segregate chromosomes to their progeny and stabilize chromosome ends. In this embodiment, the targeting vector used is a bacterial plasmid or other vector which does not replicate in yeast and includes targeting DNA and a selectable marker gene that functions in yeast. The targeting vector, which preferably has been linearized by introducing a double-strand break within the targeting DNA of the bacterial plasmid, is introduced into yeast cells. The resulting mixed population of yeast cells is maintained under conditions appropriate for homologous recombination to occur between targeting DNA and target DNA in the YAC. This is followed by selection of yeast cells stably transformed with the targeting DNA and selectable marker gene. Stable transformation of the yeast cells confers on them a selectable phenotype, such as antibiotic resistance or nutrient prototrophy, such as amino acid prototrophy or nucleoside prototrophy. Growth of yeast cells under conditions compatible with survival only of stably transformed cells is indicative of the presence of the target DNA sequence. Target DNA can be removed from the yeast cell and sequenced or manipulated, using known techniques.

The present invention also relates to vectors useful in the present method. Vectors include targeting vectors, which are generally bacterial plasmids which do not replicate in yeast and include targeting DNA and a selectable marker gene functional in yeast. They may also include a bacterial origin of replication and a selectable marker gene for selection in bacteria. YAC arm vectors useful in the present method are also the subject of the present invention. These include a yeast selectable marker gene, a bacterial origin of replication, a bacterial selectable marker gene, a yeast telomere, and one or more cloning sites at which targeting DNA is introduced or inserted into the vector. In addition, YAC arm vectors can include yeast centromere sequences and a yeast replication origin.

The present invention further relates to eukaryotic host cells, particularly yeast cells, constructed as described herein and useful for construction of YAC libraries from which a DNA fragment of interest can be identified and isolated by the claimed method. In addition, the present invention relates to DNA fragment libraries, particularly YAC libraries, constructed in such eukaryotic host cells.

The method, targeting vectors, YAC arm vectors and DNA fragment libraries of the present invention are useful for identifying and isolating a target DNA fragment, which can be genomic DNA or cDNA and can be an entire gene, gene portion or other DNA sequence. The DNA in DNA fragment libraries screened by this method can be of any type, such as mammalian (particularly human), plant, insect, avian, fish, crustacean, molluscan, viral and protozoan. For example, they can be used to identify and isolate a gene associated with a particular disease or condition, related genes within an organism's genome, and cDNA.

Further, as described herein, physically contiguous DNA sequences can be identified in a YAC library in yeast cells (or other DNA fragment library) and used to construct a physical chromosome map. That is, the present method is useful for chromosome walking. In this embodiment, a first YAC containing a target DNA fragment is isolated, using the claimed homologous recombination-based method described herein, and a terminus of the fragment is subcloned. In many instances, both termini will be subcloned in order to determine the correct direction for the walk to proceed. The terminus of the first target DNA fragment is then used as the targeting DNA present in the targeting vector, which is introduced into the YAC library. A second target YAC is isolated, which has as part of it the target DNA fragment, which partially overlaps the first target DNA in sequence. The second terminus is subcloned and used as the targeting DNA in a targeting vector introduced into the YAC library. This results in isolation of a third YAC containing a target DNA fragment, which partially overlaps the second target DNA fragment in sequence. This process results in isolation of a series of YAC containing target DNA fragments which partially overlap and can be repeated as many times as needed to construct the physical map sought. Chromosome walking can be carried out by the method of the present invention by using DNA which displays a restriction fragment length polymorphism (or RFLP) as targeting DNA in the targeting vector to screen a YAC library. A terminus of target DNA isolated in this manner is subcloned or isolated and the resulting sequence used to isolate a contiguous DNA fragment. This is repeated as often as needed to construct the physical map and, optimally, to reach a desired gene with which, for example, the RFLP is associated.

The method of the subject invention has numerous advantages over other approaches to screening DNA libraries. For example, it is possible to screen a DNA fragment library many times simultaneously. Libraries are stored as a pool of clones, thus eliminating the work to organize and screen a library that is distributed over many filter membranes. The time needed to screen a library is considerably less than that needed with conventional methods.

### Brief Description of the Drawings

Figure 1 illustrates the identification of target DNA fragments in a YAC library by the homologous-recombination selection method of the present invention. The YAC includes telomeres (arrowheads), centromere/yeast origins of replication (filled circles), and a DNA fragment; in the case of clone #3, the DNA fragment contains within it a target DNA fragment (solid rectangle).

Figure 2 illustrates targeting (homologous reciprocal recombination) to generate a YAC that is marked for selection.

Figure 3 illustrates selection by homologous recombination of a DNA clone from a DNA YAC library using one-step gene disruption.

Figure 4 is a map of plasmid p184DLARG. B: BamHI; Sm: SmaI; P: PstI; ARG4: yeast ARG4 gene (arrow indicates direction of transcription); Cm: chloramphenicol resistance gene; ORI (pACYC184): Origin of replication from pACYC184; -------: hypothetical targeting sequence inserted into cloning site.

Figure 5 is a restriction enzyme and Southern blot analysis of clones selected by targeting with human epsilon- and beta-globin sequences.

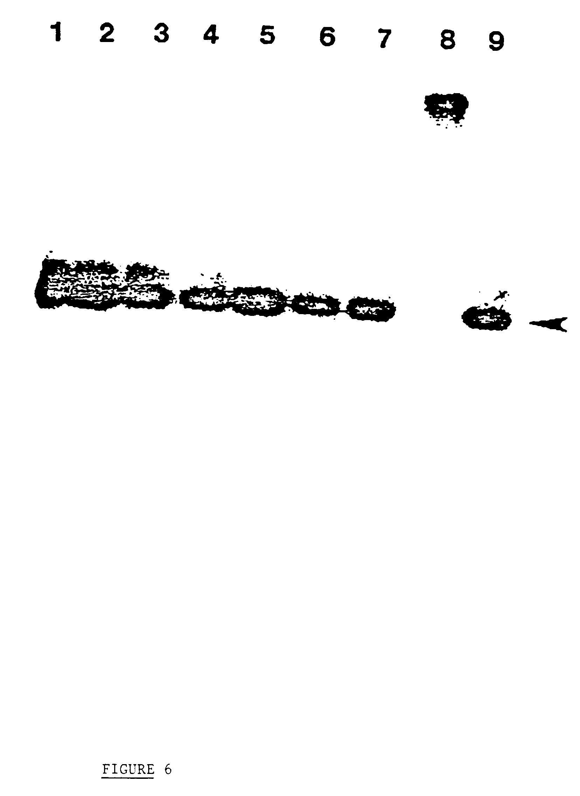

Figure 6 is DNA from eight colonies isolated by screening with fragment 8A. Lanes 1-4: clones 8A.1, 8A.2, 8A.3 and 8A.4; Lane 5: plasmid p184-8A. Lanes 6-7: clones 8A.5 and 8A.6; Lane 8: an example of DNA from an isolated colony which does not show the unit-length-linear band; Lane 9: clone 8A.11. 1 microgram of total yeast DNA was loaded in lanes 1-4 and 5-9. 2 nanograms of plasmid p184-8A was loaded in lane 5. The electrophoreased DNA samples (all digested with KpnI) were transferred to a nylon membrane and hybridized with a 32-P labeled ARG4 DNA probe. The arrow marks the position of the unit-length-linear band at 8.3 kb.

Figure 7 is DNA from each of the positive clones digested with XhoI and with either KpnI (for those isolated by screening with fragment 8A) or AvaI (for those isolated by screening with fragment 10B). Samples were electrophoresed on a 1% agarose gel, transferred to a nylon filter, and hybridized with ³²P labeled pBR328 (Boehringer Mannheim Biochemicals, Indianapolis, IN). Lanes 1-7: clones 8A.1, 8A.2, 8A.3, 8A.4, 8A.5, 8A.6, 8A.11 (all isolated by screening with fragment 8A); lanes 8-10: clones 10B.6, 10B.29, 10B.41 (isolated by screening with fragment 10B).

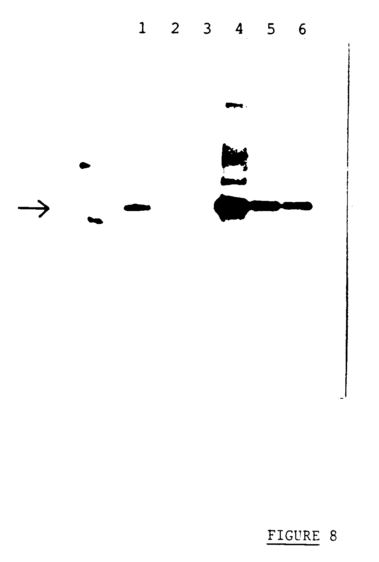

Figure 8 shows analysis of YAC DNA for presence of unit-length-linear fragments hybridizing to an ARG4 DNA probe: Lane 1: EcoNI digest of plasmid p184DLARG/PCRF.5, which contains the 852 base pair PstI fragment from the human ADA locus clones into the PstI site of 184DLARG. 1 nanogram of digested plasmid DNA was loaded; Lanes 2-3: empty (no samples loaded); Lanes 4-6: EcoNI digested YAC DNA (approximately 1 microgram) from candidate transformants 184ADA.B, 184ADA.C and 184ADA.D. The electrophoresed samples were transferred to a nylon membrane and hybridized to a 32-P labeled fragment of ARG4 DNA. The arrow indicates the position of EcoNI linearized plasmid p184DLARG/PCRF.5 (5.2 kb).

Figure 9 is a schematic representation of one embodiment of the present homologous recombination method, in which a YAC containing target DNA is identified using recombination with a linear yeast plasmid.

### Brief Description of ATCC Deposits

The following deposits have been made at the American Type Culture Collection (June 28, 1990) under the accession numbers indicated. These deposits have been made under the terms of the Budapest Treaty.
1. Saccharomyces cerevisiae strain TD7-16d, ATCC No. 74010.
2. Plasmid p184DLARG, ATCC No. 40832.

### Detailed Description of the Invention

The present invention is based upon Applicant's discovery that the process of homologous recombination which occurs in eukaryotic cells can be used for the purpose of screening DNA fragment libraries constructed in eukaryotic cells and identifying and isolating a DNA fragment of interest, referred to as a target DNA fragment.

The present invention is a method of isolating a DNA fragment of interest, referred to as a target DNA fragment, from a DNA library constructed in a eukaryotic host in which genetic recombination occurs by homologous recombination. The target DNA fragment is generally present in a larger fragment contained in the eukaryotic host cell. The DNA used to construct the DNA libraries may be cDNA or genomic DNA which is of human or other origin. A target DNA fragment is identified by the present method by introducing into the DNA fragment library a non-replicating targeting vehicle which contains targeting DNA and an appropriate selectable marker gene and identifying eukaryotic host cells in which homologous recombination occurs between target DNA and targeting DNA. Homologous recombination results in stable integration of targeting DNA and the selectable marker gene into DNA in host cells, which are identified on the basis of a selectable phenotype conferred as a result of stable transformation of host cells with the selectable marker gene. For example, they are identified on the basis of their ability to grow under conditions (e.g., in the presence of a drug or in the absence of an essential nutrient) incompatible with growth of host cells in which stable integration has not occurred.

The DNA library used in the present method is a population of eukaryotic host cells, such as yeast cells, containing a unit, such as an artificial chromosome, which includes a DNA fragment insert and is replicated in the host cells. The DNA library is screened for DNA fragment insert(s), present in the artificial chromosome, all or a portion of which is a target DNA fragment, by introducing into the eukaryotic host cells a targeting vehicle, such as a bacterial plasmid, which is non-replicating in the eukaryotic host cells and includes a targeting DNA sequence (i.e., a DNA sequence homologous, at least in part, to the target DNA) and a selectable marker gene useful for selection in the host cell. Host cells containing the targeting vehicle are cultured under conditions appropriate for homologous recombination between the targeting DNA sequence and target DNA to occur. Host cells stably transformed with the selectable marker are subsequently identified (i.e., by identifying host cells able to grow under conditions under which non-stably transformed cells cannot grow, and die).

In a specific embodiment of the present invention, which is exemplified by the Examples which follow, the DNA library is a population of yeast cells which contain artificial chromosomes carrying a DNA fragment insert and host cells containing target DNA are identified and isolated from this YAC vector library.

A targeting vehicle, such as a bacterial plasmid, which is non-replicating in yeast is introduced into the population of host yeast cells containing the DNA YAC library. The bacterial plasmid includes a targeting DNA sequence which is homologous, at least in part, to target DNA of interest and a selectable marker gene that functions in yeast. Preferably, the targeting plasmid is cut with a restriction endonuclease that introduces a double-strand break within the targeting DNA sequence, thereby linearizing the bacterial plasmid and providing DNA ends which are recombinogenic, to stimulate the process of homologous recombination with the YAC sequences. The efficiency of homologous recombination is, as a result, increased. Because the plasmid is non-replicating in yeast, stable transformation with the selectable marker can only proceed by homologous recombination.

The resulting host yeast cell population, which includes stably transformed host yeast cells (i.e., those in which the plasmid, including the selectable marker gene, has been stably integrated by homologous recombination into DNA already present in host cells prior to introduction of the targeting vehicle) and non-stably transformed host yeast cells, is cultured under conditions such that only stably transformed yeast cells are able to grow. In a correctly targeted event, the entire plasmid is stably incorporated in the host yeast cells by homologous recombination between the targeting DNA sequence of the plasmid and homologous sequences (i.e., target DNA fragments) in the YAC. In other embodiments, such as when a linear targeting plasmid is used, it is not necessary, however, for the entire plasmid to become stably incorporated, as long as homologous recombination occurs to an extent sufficient to introduce a selectable marker gene into DNA already present in the host cells, such as in a YAC. Only those few host yeast cells which contain a target DNA fragment(s) and have thereby undergone homologous recombination with the targeting plasmid are able to grow under the conditions used (e.g., in antibiotic-containing medium or medium lacking a nutrient essential to non-stably transformed cells), due to the introduction of the yeast-selectable marker gene contained on the targeting plasmid. They are identified on the basis of the selectable phenotype conferred by stable transformation of the selectable marker gene.

To prevent homologous recombination events between the plasmid-borne yeast-selectable marker gene and homologous sequences in the host yeast cells, it is preferable that host cell sequences homologous with targeting vector sequences have been deleted or almost entirely deleted from the genome of the host yeast strain before it is used for the YAC vector library. Alternatively, host cell sequences homologous with a yeast-selectable marker gene on the incoming targeting plasmid can be retained as a mutated, non-functional portion of the yeast chromosome. If this approach is used, however, more positive scores for homologous recombination will have to be screened to ensure that homologous recombination events which occur took place between the targeting DNA sequence on the bacterial plasmid and the target DNA sequence present on the YAC.

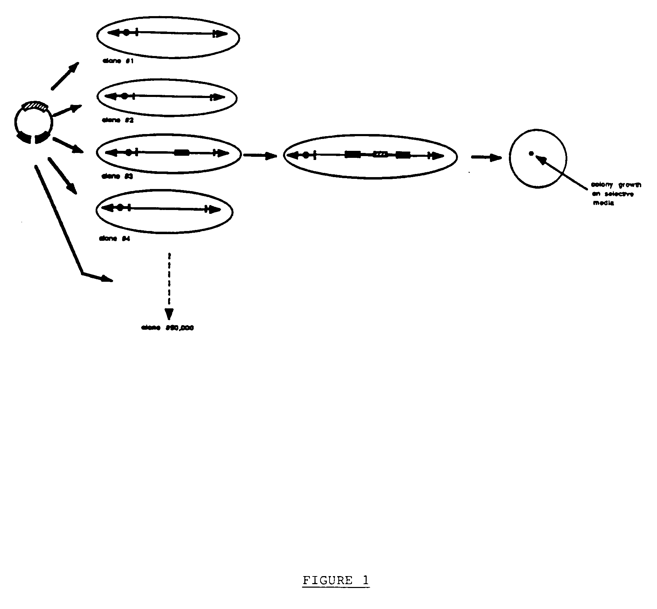

Figure 1 illustrates schematically the isolation of target DNA fragments from a YAC vector library by the method of the present invention. The targeting plasmid on the far left is introduced into a population of yeast cells (ovals), each of which contains a DNA YAC containing a different DNA fragment. The plasmid includes a selectable marker gene for selection in yeast (diagonally lined section) and a targeting DNA fragment (solid section) in which a double strand break has been introduced. In this example, one host yeast cell (#3) contains a DNA fragment in a YAC that is homologous to a sequence carried on the targeting plasmid (solid sections on clone #3). Recombination between these two sequence occurs, resulting in the stable integration of the selectable marker carried on the plasmid into the yeast chromosome (YAC). The resulting population of cells is grown under conditions appropriate for selection of host yeast cells stably transformed with the selectable marker gene. For example, they are plated on appropriate selective media, such as nutrient-deficient media. Only those cells in which the selectable marker gene functions grow. Growth of cells under these conditions is indicative of the presence of a target DNA fragment. Although YAC are exemplified herein, other yeast vectors, such as YCp vectors (YCp50, YCp19) can be used to construct a DNA library.

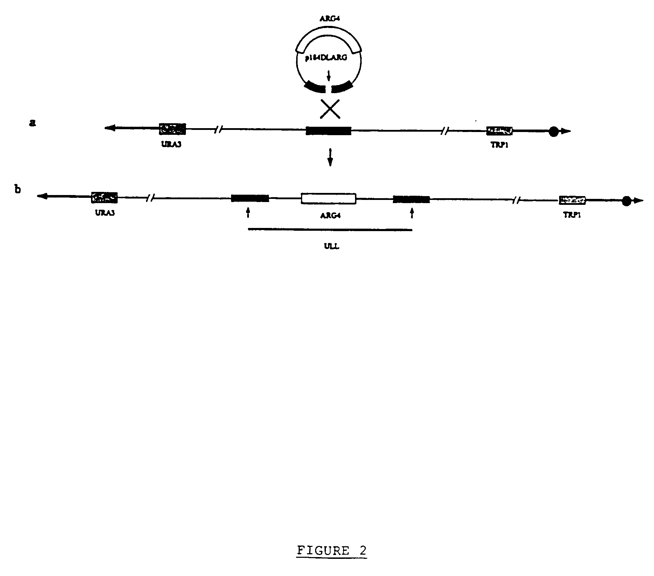

The general scheme for selection of a target DNA fragment from a DNA YAC library is shown in Figure 2. Figure 2 illustrates the integration of a targeting plasmid (p184DLARG) carrying a selectable marker (the yeast ARG4 gene; open box) and a segment of DNA that is homologous to a sequence in the DNA YAC library (targeting DNA; solid arcs on plasmid). The thin lines represent an insert of human or other (non-yeast) DNA propagated as a yeast artificial chromosome (YAC). The solid black box is the target DNA fragment, a sequence of DNA which is a portion of YAC DNA present in a DNA clone, found in the library, that is homologous to the targeting sequence. The remaining portions of the DNA YAC are comprised of the YAC vector arms: the thick lines represent plasmid vector sequences for replication and selection in bacteria. The shaded boxes represent genetic markers used for selection in yeast (yeast selectable markers URA3 and TRP1). The solid arrowheads and circle represent telomeres (TEL) and a centromere/ yeast replication origin (CEN/ARS), respectively. Figure 2a depicts the targeting DNA (present in the targeting vector) aligning with the target DNA fragment in the YAC. Figure 2b depicts the product of homologous recombination between the targeting DNA and target DNA fragment. The targeting plasmid has been cut uniquely in the targeting DNA, at the site corresponding to the vertical arrow in the target sequence. ULL indicates the unit length linear restriction fragment that results from duplication of the target sequence (and the restriction site) on the YAC. As described in Example I, a ULL can be generated only if integration occurs into a DNA sequence that contains the restriction enzyme site in question and contains sufficient homology surrounding that site to allow resynthesis (by repair) of the restriction enzyme site on the targeting plasmid. Candidate clones that display a ULL are assumed to be homologous recombination events and are analyzed further.

In another embodiment of this method, a yeast-selectable marker gene on the incoming targeting DNA molecule can be a bacterial gene engineered to be expressed in yeast and confer drug resistance, e.g., the CAT or neo genes from Tn9 and Tn903, or bacterial amino acid or nucleoside prototrophy genes (e.g., the E. coli argH, trpC, and pyrF genes).

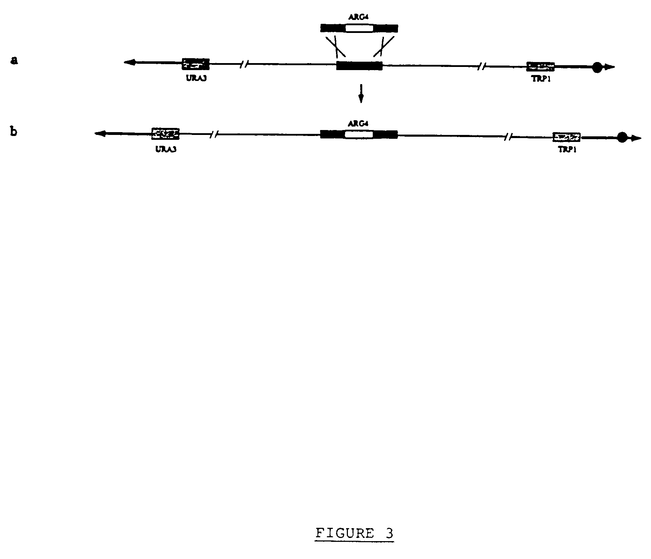

In another embodiment of the method of the present invention, the targeting vector is a linear DNA fragment which includes a targeting DNA sequence homologous to a target DNA fragment to be identified and/or isolated from the YAC library. In this embodiment, a selectable marker gene is inserted into the targeting DNA, producing a targeting DNA sequence which includes two non-contiguous domains. This embodiment is described in detail in Example II and represented schematically in Figure 3. The targeting vector, which is a linear sequence which does not replicate in yeast, is transformed into the pooled DNA YAC library, as described in Example I. Homologous recombination occurs between the targeting DNA and the target DNA fragment.

In addition to the above-described embodiment, other approaches to introducing targeting DNA into host cells can be used. For example, targeting DNA can be present on a replicating yeast linear plasmid (Murray, A.W. and Szostak, J.W., Nature 305:189-193 (1983)) in a yeast strain of mating type opposite to that of the host strain used for the library. The linear plasmid has selectable markers flanking the targeting DNA sequence (i.e., one at each end of the targeting DNA); both markers are different from those used in the construction of the YAC library and can be selected against (i.e., negatively selectable markers, such as LYS2, URA3 or CYH2). Homologous recombination between two linear molecules produces two linear molecules, each of which is a hybrid of the two parental molecules. In this embodiment, in which recombination occurs between the targeting linear plasmid and a library YAC, each of the two recombination products is a YAC and each carries one of the two negatively selectable markers, allowing for differential selection of the two recombination products.

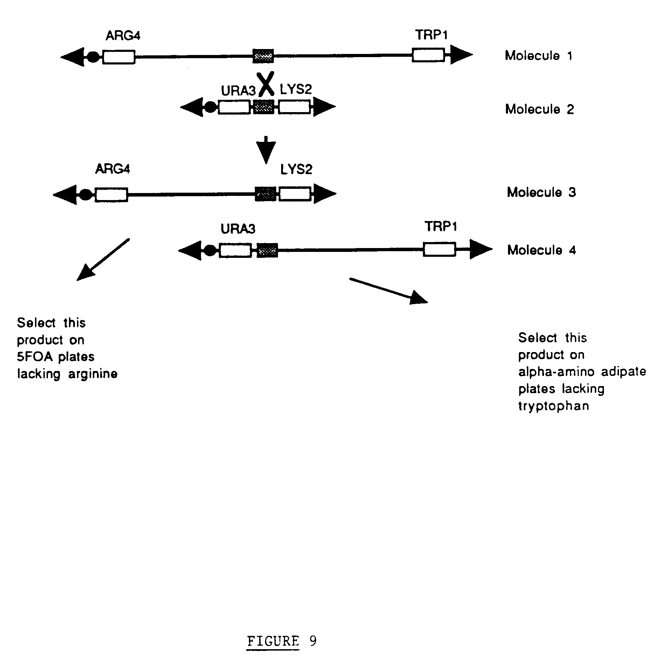

The basis of this differential selection is illustrated in Figure 9. Filled circles, arrowheads, and open rectangles represent centromeric, telomeric, and marker gene sequences, respectively. The shaded boxes represent targeting or target sequences. URA3⁺ cells can be selected against (killed) by growth on media containing the nucleoside analog 5-fluoro-orotic acid (5FOA), while LYS2⁺ cells can be selected against by growth on media containing the amino acid analog alpha-amino-adipic acid (αaa). Molecule 1 is a target YAC constructed in a vector system using ARG4 and TRP1 as selectable markers (phenotype arg⁺ trp⁺ 5FOA^{R}αaa^{R}). Molecule 2 is a linear targeting plasmid in which the targeting sequence is flanked by URA3 and LYS2 (phenotype arg⁻ trp⁻ 5FOA^{S} αaa^{S}). The phenotype of cells harboring molecules 1 and 2 in an unrecombined form is arg⁺ trp⁺ 5FOA^{S} αaa^{S}. Molecules 3 and 4 are the products of recombination between Molecules 1 and 2, resulting from a cross-over between the targeting and target sequence. The phenotype of Molecule 3 is arg⁺ trp⁻ 5FOA^{R} αaa^{S}, and can be selected for by growth on 5FOA plates lacking arginine. The phenotype of Molecule 4 is arg⁻ trp⁺ 5FOA^{S} αaa^{R}, and can be selected for by growth on αaa plates lacking tryptophan. Thus, cells containing one or both non-recombinant molecules, as well as cells containing either of the recombinant products can be differentially selected (cells harboring only one or the other recombinant product arise by random loss events).

In such a scheme, the yeast cells harboring the targeting linear plasmids are mated to all members of the library and maintained under conditions favorable for spontaneous or induced homologous recombination (induced by, for example, meiosis or ultraviolet irradiation). Recombinant target YACs are selected by virtue of the unique phenotypes of the recombination products resulting from homologous recombination between the targeting sequence on the linear plasmid and YAC molecules harboring a suitable target sequence. Each of the two product YACs is truncated at the position of the target DNA sequence, and the differential selection is used to isolate the two products separately. In order to isolate the two products of a single event, yeast cells harborings YACs and linear targeting plasmids are preferably plated or gridded out prior to selection for recombinants. Selection is accomplished by replica plating onto the appropriate selective plates.

In this embodiment, the relative orientation of the targeting sequence with respect to the two (negatively) selectable markers on the linear targeting plasmid is important. Recombination between a target YAC and only one of the two orientations of targeting linear plasmid will give rise to a stable recombinant (i.e., a recombinant with one and only one centromere). YAC molecules with two centromeres show frequent breakage and unstable phenotypes and YAC molecules with no centromere are highly unstable by virtue of segregation bias. In a preferred embodiment, linear targeting plasmids are constructed with the targeting sequence present in both orientations and introduced into the library in separate matings.

As an alternative to mating to introduce linear targeting plasmids into the library, linear targeting plasmids can be introduced into host cells containing YACs by transformation, essentially as described in Example I.

In another embodiment, a yeast replicating plasmid carrying a targeting sequence can be constructed in such a manner that the targeting DNA and a selectable marker (SM1) can be freed from the yeast replicon by natural or induced recombination events, and such that the replicon itself can be selected against by virtue of a negatively selectable marker (SM2), such as URA3, LYS2 or CYH2. Examples of inducible recombination systems which can be engineered to function for this purpose are the flp mediated recombiantion pathway of the yeast 2-micron plasmid and the cre-lox recombination system of bacteriophage P1. The plasmid is introduced into a yeast strain of mating type opposite to that of the host strain used for the library. After mating to all members of the YAC library, the targeting DNA sequence and selectable marker are released as a nonreplicating molecule and the selectable marker can only be stabilized by homologous recombination with a YAC harboring a suitable target DNA sequence. The targeted recombinants are selected by plating onto media which selects for SM1 and against SM2.

As an alternative to mating to introduce the plasmid described in the preceding paragraph, the plasmid can be introduced by transformation, eseentially as described in Example I, followed by the induction step to free the targeting substrate from the yeast replicon.

### Identification and Isolation of a Target DNA Fragment Using Homologous Recombination

The above-described embodiments of the present method are useful to identify and isolate any target DNA fragment, which can be an entire gene, a gene portion or other nucleotide sequence. For example, a gene of interest, such as a β-globin gene or adenosine deaminase gene, can be identified in a DNA fragment library using the claimed method and, if desired, isolated from host cells by known methods. Identification of target DNA fragments by the present method is described in detail in Examples I, III and IV.

### Homologous-Recombination Chromosome Walking

The method of the present invention, by which a target DNA fragment is isolated from a DNA library, is useful for isolating physically-contiguous DNA segments from a DNA YAC library in order to construct a physical chromosome map. That is, when used iteratively, each time with targeting DNA derived from a YAC which overlaps with and extends beyond a previously identified region, it is a method for chromosome walking. In the present method of chromosome walking, a target DNA fragment present in a YAC is isolated, as described above. A terminus of this first target YAC fragment is subcloned into a plasmid vector. The terminus of the first DNA fragment is, thus, used as a second targeting DNA sequence, which is introduced into host yeast cells containing a DNA YAC library. The terminus of the first DNA fragment, which is contiguous to the first target DNA sequence, in turn becomes the second targeting DNA sequence. As used herein, the term contiguous includes sequences which are immediately adjacent to the first target sequence and those nearby or in proximity to the first target sequence (i.e., separated from the first target sequence by intervening nucleotide(s)). This second targeting DNA sequence should not have any homology with the first targeting DNA sequence, so that when it in turn is incorporated in a YAC at a point of homology with a second DNA clone, the second DNA clone selected will have a different terminal DNA sequence. The terminal subfragment from the second DNA clone is used to isolate the next (i.e., the third) DNA clone. Each successive DNA clone is isolated by virtue of its homology with the termial subfragment of the previously isolated DNA clone. A series of overlapping clones is obtained by repeating this process; the process is repeated as needed to construct the physical map desired. The successive recovery of terminal DNA fragments allows rescreening the same library or a second library for overlapping clones.

In one embodiment of the present invention, chromosome walking is carried out in order to determine the chromosomal location of a gene of interest, such as a gene which causes a disease, by using a DNA fragment displaying a RFLP genetically linked to the gene of interest, or a DNA fragment contiguous with the RFLP, as targeting DNA in the targeting vector. A targeting vector, such as a bacterial plasmid, which includes the RFLP-displaying DNA or fragment contiguous to the RFLP-displaying DNA, as targeting DNA and a selectable marker gene, is introduced into a human DNA YAC library. Homologous recombination between the targeting DNA and a target DNA fragment in the library results in the first step in walking to the gene of interest. A YAC containing the target DNA fragment is identified in this way. One terminus or both termini of the target DNA fragment is used as targeting DNA in a targeting vector to rescreen the same library or screen a second library, as described above. Also as described above, this is repeated, each time using a terminus of the target DNA fragment isolated in the previous step as targeting DNA. This continues until the gene of interest is identified or the desired physical map is completed.

### Host Cell Types and Characteristics

The method is described herein with particular reference to screening YAC DNA libraries constructed in yeast cells through the use of targeting DNA sequences present in bacterial plasmids. It is to be understood, however, that this is merely for purposes of exemplification and that the present method can be carried out using other host cell types, provided that genetic recombination between vector-borne DNA and DNA already present in the host cell occurs by homologous recombination and that an appropriate targeting vector is available.

Appropriate eukaryotic host cells include those which normally (as they occur in nature) undergo genetic recombination essentially exclusively by homologous recombination (e.g., Saccharomyces cerevisiae, Schizosaccharomyces pombe). As used herein, the term essentially exclusively means that homologous recombination occurs without significant levels of non-homologous recombination under the conditions used. Homologous-recombination selection of DNA clones could be utilized as a selection method in the cells of any organism in which 1) a suitable DNA cloning system exists and 2) the cells can be manipulated or induced, by genetic engineering or genetic manipulation, to perform recombination which is predominantly based on DNA sequence homology, or in which the targeting DNA can be treated in such a manner that it engages in homologous-recombination as its preferred mode of recombination. With these criteria met, one skilled in the recombinant DNA arts could perform homologous-recombination selection of DNA clones from a DNA library. Such organisms may include, but are not limited to, Schizosaccharomyces pombe, Drosophlla melanogaster, Homo sapiens, Mus musculus and Spodoptera frugiperda.

Saccharomyces cerevisiae is a preferred host organism for the selection of DNA clones using homologous-recombination because of its ability to route transforming DNA carrying double-strand breaks into a recombination pathway based virtually exclusively on DNA sequence homology.

Certain characteristics of host cells in which DNA fragment libraries are constructed should be considered and possibly modified to optimize use of such cells in the present method, such as by decreasing non-targeted events and, thus, increasing the efficiency of the method. For example, as described below, it might be necessary to remove selectable markers present in the targeting vector from host yeast cells and to construct targeting vectors in such a manner that they include no sequences homologous with those in the vector sequences used in the propagation of the DNA library.

As described below, it has been determined that the selectable marker gene(s) chosen for the targeting vector should not normally be present in the host yeast genome or should be deleted from the normal chromosomal position(s) in the host yeast strain. Without this modification of the host strain, recombination events between the selectable marker and the yeast genome would occur at a higher rate. For near-complete (> 99%) coverage of the human genome, a DNA YAC library with an average fragment size of 300 kb would consist of approximately 50,000 members (Maniatis, T. et al., Molecular Cloning-A Laboratory Manual, pg 271, Cold Spring Harbor Laboratory Press, Cold Spring Harbor, New York, 1982). In order to isolate sequences that are represented only once in such a library, the ratio of targeted to non-targeted events should approximate or exceed 50,000 to 1, for at this ratio one incorrect (non-targeted) clone will be isolated for every correct clone. In most cases, however, sequences in this library will be represented 3-5 times, and a ratio of 10,000-17,000 to 1 would be adequate. Non-targeted events result from recombination between the targeting plasmid and regions of homology in the yeast cell, and can be minimized by decreasing the extent of such homology. As described in Example V, it was determined that deleting the chromosomal copies of selectable marker genes present on the vectors used is desirable because it reduces the occurrence of non-targeted events. As described in Example V, these results indicate that the selection of a targeted clone (target DNA fragment) from a DNA YAC library is feasible and particularly efficient in host yeast cells that carry no homology with selectable markers present on targeting vectors.

Non-targeted events might also occur as a result of homologous recombination between sequences on the targeting vector, such as the bacterial plasmid origin of replication or drug resistance marker, and homologous sequences on the YAC vector arms used to construct the DNA library. This homology can be minimized by constructing the targeting vector using a drug resistance marker that is not present in the YAC vector, and by using a bacterial plasmid origin of replication that is divergent from or non-homologous to the origin present on the YAC vector arms. The results described in Example V also place the frequency of non-homologous recombination at approximately 0.003% (1 in 30,729), consistent with the invention described in this application. It was possible to select yeast cells carrying homology to the targeting vector even when only 1 in 10,000 of the cells transformed had such homology. In fact, at this dilution targeted events were isolated multiple (four) times, indicating that a clone represented once in a library of 40,000 clones could be isolated.

### Targeting Vectors

Targeting vectors useful in the method described herein are also the subject of the present invention. Targeting vectors of the present invention have two key characteristics: they are non-replicating in the host cell in which the DNA fragment library is constructed and they include a DNA sequence, referred to as targeting DNA, which is homologous to a target DNA fragment which, for the purposes of the invention, is a DNA fragment comprising all or a portion of a desired clone to be identified in and isolated from the DNA library. Targeting vectors will generally be bacterial plasmids of the YIp class, particularly in those cases in which yeast cell hosts are used. Vectors appropriate for other types of cell hosts can also be constructed using known techniques.

Sequences used as targeting DNA in the targeting vector can be entirely homologous to the target DNA fragment, although they need not be. They need be only sufficiently homologous that under the conditions used, genetic recombination between vector-borne DNA introduced into the cells and DNA in YAC in the cells occurs by the host cell recombination pathway or process. Preferably, a double strand break is introduced into a targeting DNA sequence. Alternatively, a double strand gap can be introduced. The free ends adjacent to the break or gap can be modified to prevent recircularization (e.g., by phosphatase treatment of the ends of the DNA or by creating non-complementary ends by using two different restriction enzymes).

In addition to targeting DNA, targeting vectors include a selectable marker gene that functions in yeast, an origin of replication and a selectable marker that functions in bacteria (e.g., E. coli.). The selectable marker gene is one which is functional (makes selection of transformed cells possible) in the host cell type used for DNA fragment library construction. The choice of the yeast selectable marker gene can be made from among many various endogenous yeast gene loci, e.g., ARG4, LEU2, HIS3, HIS4, THR1, URA3, TRP1, LYS2, ADE2, ADE8, and MET2. Alternatively, the yeast selectable marker may be a marker gene that is not endogenous to the yeast genome, but is a foreign gene that confers a selectable phenotype, e.g., a bacterial gene engineered to be expressed in yeast and confer drug resistance on the yeast cells (such as the CAT or neo genes from transposons Tn9 and Tn903, respectively) or amino acid or nucleoside prototrophy (such as E. coli argH, trpC, or pyrF genes). Other selectable marker genes useful for this purpose include genes which confer tolerance to metal ions (e.g., the CUP1 gene, which confers resistance to copper ions), genes which confer an ability to progress through the cell cycle in cells with a mutant phenotype and genes which result in expression of a cell surface marker.

The suitable selectable marker genes for selection in bacteria include the genes encoding resistance to the antibiotics, kanamycin, ampicillin, tetracycline, spectinomycin, streptomycin, erythromycin, or any other marker, including genes encoding biosynthetic enzymes for which auxotrophic bacterial hosts exist.

Bacterial origins of replication may be derived from a variety of source, including p15A (exemplified by the origin of plasmid pACYC184), ColE1, phage M13, phage f1, phage Lambda, or any other replicon that one trained in the art would recognize as providing an equivalent function.

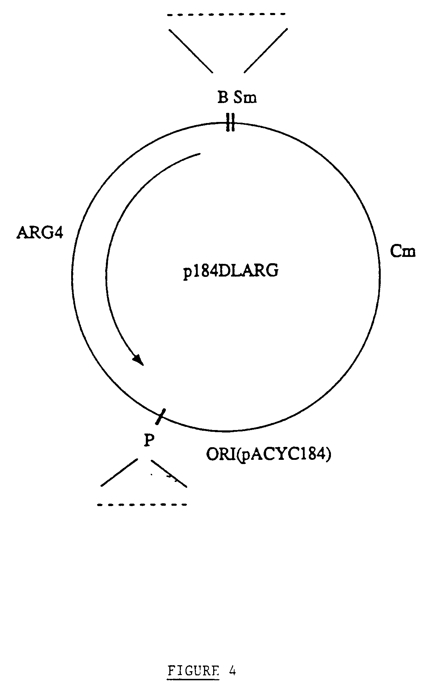

Vectors constructed and used to screen YAC DNA libraries are described in detail in Example I and represented schematically in Figure 4. Targeting plasmid p184DLARG contains a selectable marker functional in yeast (ARG4) and a bacterial origin of replication (derived from pACYC184).

Targeting DNA molecules are not limited to molecules of the Yip class. The targeting DNA can be a fragment of DNA purified from a larger plasmid, with such a plasmid constructed in such a manner that the desired targeting sequence is interrupted by, among other sequences, a bacterial or yeast replicon. The plasmid is also constructed such that upon cleavage with a restriction enzyme that will release the replicon from the inner section of the targeting sequence, a yeast selectable marker remains covalently linked to the outer two ends of the targeting sequence.

Alternatively, a selectable marker and a targeting sequence can be ligated together in vitro and ligation products consisting of one copy of the targeting sequence and one copy of the selectable marker (or multimers consisting of alternating targeting and selectable marker sequences in a uniform orientation) are purified. These ligation products are circularized in vitro and cleaved with a restriction enzyme to introduce a double-strand break or gap in the targeting sequence and leaving the selectable marker intact.

Finally, the two halves of a targeting sequence can be ligated to a selectable marker in a single three-way ligation in vitro to generate a targeting molecule suitable for transformation.

The present invention is illustrated by the following Examples, which are not intended to be limiting in any way.

### Methods Used Herein

Unless otherwise noted, methods for plasmid purification, restriction enzyme digestion of plasmid DNA and gel electrophoresis, use of DNA modifying enzymes, ligation, transformation of bacteria, transformation of yeast by the lithium acetate method, preparation and Southern blot analysis of yeast DNA, tetrad analysis of yeast, preparation of liquid and solid media for growth of E. coli and yeast, and all standard molecular biological and microbiological techniques can be carried out essentially as described in Ausubel et al. (Ausubel, F.M. et al., Current Protocols in Molecular Biology, Greene Publishing Associates and Wiley-Interscience, New York, 1987).

### EXAMPLE I: SELECTION BY HOMOLOGOUS-RECOMBINATION OF A TARGETED DNA CLONE FROM A DNA YAC LIBRARY

Plasmid pYAC4 (ATCC #67380) was used to construct a library of human genomic DNA. Human DNA was isolated from white blood cells (D. Burke, Ph.D. Thesis, Washington University, St. Louis, MO., 1988), partially digested with EcoRI and ligated to pYAC4 arms digested with EcoRI and BamHI.

The ligation mixture was then used to transform yeast host strains, either MGD131-10c or IV-16d, using the spheroplast method (Burgers, P.M.J. and Percival, K.J., Analytical Biochemistry 163:391-397 (1987)). (The construction of host strains MGD131-10c and IV-16d with the appropriate marker deletions is described at the end of the Example.) Since the pYAC4 vector carries the yeast selectable markers TRP1 and URA3, transformants can be selected for by growth on plates lacking tryptophan and uracil. 11,625 YACs with an average size of 190 kb (0.73 human genome equivalents) are individually grown in the wells of 96-well microtiter plates; 0.1 ml was taken from each well and pooled in three subpools of approximately 4,000 clones each. For each subpool, an equal volume of 30% glycerol was added and the subpool was aliquoted and frozen at -70°C.

For a library comprising 73% of one genome, and assuming equal representation of all clones, the probability that it contains any one specific human DNA sequence is just over 0.5. The probability that one of six different fragments of DNA is represented in the library is 1-(0.5)⁶, or 0.98.

The construction of the targeting plasmid p184DLARC is described below and illustrated in Figure 4. It carries the yeast ARG4 gene (Beacham, I.R. et al. (1984) Gene 29:271-279) as a selectable marker, and its bacterial origin of replication is derived from pACYC184 (Chang, A.C.Y. and Cohen, S.N. (1978) Journal of Bacteriology 134:1141-1156.), which shares only limited sequence homology to the pBR322 origin used on pYAC4. The entire chromosomal copy (a 2.0 Kb HpaI DNA fragment) of ARG4 has been deleted in the library host strains IV-16d and MGD131-10c. The 2.2 kb BclI-ClaI fragment from pACYC184 (Chang, A.C.Y. and Cohen, S.N., (1978), Journal of Bacteriology 134:1141-1156.) containing the p15A origin of replication and the chloramphenicol resistance gene was ligated to BamHI-AccI digested pMLC28 (a derivative of pSDC12 carrying the pUC18 multiple cloning site; Levinson et al., J. Mol. Appl. Gen., 2:507-517 (1984); plasmid pUC18 (ATCC #37253) can substitute for pMLC28 in the construction of p184DLARG described here). BamHI and AccI cut this plasmid one time each, in the polylinker. The ligation mixture was digested with SacI and HindIII, which cut in the pMLC28 polylinker, and the digested DNA was treated with T4 DNA polymerase to generate blunt ends. The DNA was ligated under dilute conditions to promote circularization, and the ligation mix was treated with the restriction enzyme AvaII (to linearize any parental molecules) prior to transformation into bacteria. One plasmid, p184DL, carrying only the sequences contained within the larger of the two BclI-ClaI fragments of pACYC184 and a permuted version of a portion of the pMLC28 polylinker was identified. Plasmid pHpa5 (provided by N. Schultes and J. Szostak; Department of Molecular Biology, Massachusetts General Hospital, Boston, MA 02114) carries the ARG4 gene as a 2.0 Kb HpaI fragment inserted into the HincII site of pMCL12 (a derivative of pSDC12 carrying the pUC12 multiple cloning site). Levinson et al., J. Mol. Appl. Gen., 2:507-517 (1984). This plasmid was cut at the PstI and SmaI sites flanking the ARG4 insert, and the ARG4 fragment was ligated to PstI-SmaI cut p184DL. A plasmid carrying a single copy of the ARG4 gene inserted in the orientation shown in Figure 4 was isolated and designated p184DLARC. Figure 4 is a map of plasmid p184DLARG.

Genomic fragments for tyrosine hydroxylase (chromosome 11), metallothionein II pseudogene (chromosome 4), anonymous DNA markers D16S3 and D16S37 (chromosome 16), and a 1.9 kb HindIII fragment located 5' of the epsilon globin gene (chromosome 11) were subcloned into p184DLARG and used for selection of clones by recombination from a YAC library. With the exception of the tyrosine hydroxylase gene fragment, all of the fragments were blunt ended by treatment with T4 DNA polymerase and ligated to SmaI cut p184DLARG. The tyrosine hydroxylase gene fragment was cloned into the BamHI site of p184DLARG. A 1.3 kb HpaI-BamHI fragment from the 5' end of the beta globin gene (chromosome 11) was blunt-end ligated to the same 2.2 kb BclI-ClaI fragment used to construct p184DLARG. The beta- and epsilon-globin fragments are 1.3 and 1.9 kb fragments, respectively, from the human beta-hemoglobin locus on chromosome 11. The beta-globin fragment (ATCC #39698) was subcloned from pHU5'beta (Treco, D., et al., Mol. Cell. Biol., 5:2029-2038, 1985), and includes sequences from positions 61,338 (HpaI site) through 62,631 (BamHI site) in the Genbank HUMHBB sequence. This fragment includes the 5' end of the human beta-globin gene. The AvaII site at Genbank map position 62,447 was used to introduce a double-strand break for targeting, leaving 1.1 and 0.18 kb of homology on either side of the break. The 5' epsilon-globin probe (ATCC #59157), is a HindIII fragment and includes sequences centered approximately 15 kb 5' to the epsilon-globin gene (ATCC #59157), from positions 3,266 through 5,172 in the Genbank HUMHBB sequence. The ApaI sites at map positions 4,361 and 4,624 were used to create a 0.26 kb double-strand gap for targeting, leaving 1.1 and 0.5 kb of homology on either side of the gap.

Properties of the remaining four genomic DNA fragments are as follows: tyrosine hydroxlase (chromosome 11; 2.3 kb BamHI fragment; ATCC #59475; double-strand break made with HindIII, 0.6 kb from end); metallothionein pseudogene (chromosome 4; 2.8 kb HindIII-EcoRI fragment; ATCC #57117; double-strand break made with NdeI, 0.4 kb from end); anonymous DNA marker D16S3 (chromosome 16; 1.5 kb HindIII fragment; ATCC #59447; double-strand break made with ApaI, 0.75 kb from end); D16S37 (chromosome 16; 2.3 kb HindIII fragment; ATCC #59189; double-strand break made with ApaI, 0.95 kb from end).

Each targeting plasmid was linearized with a restriction enzyme that cuts within the human DNA (the targeting DNA) and 20 µg of digested DNA was used to transform the pooled library. Equal volumes of the three library subpools were thawed, mixed and inoculated into CM -ura - trp medium containing 40 µg/ml each of kanamycin and ampicillin. This culture was grown overnight at 30°C with vigorous shaking and harvested at a density of 1.86 x 10⁷ cells/ml. The cells were transformed using the lithium acetate method (Ausubel, F.M. et al. Current Protocols in Molecular Biology, Supplement 5, Green Publishing Associates and Wiley-Interscience, New York 1987). 20 µg of plasmid cut within the human DNA was used to transform 7 x 10⁸ cells in a volume of 0.2 ml, and the entire transformation mix was spread onto the surface of eight selective plates (complete minimal media lacking uracil, tryptophan, and arginine) and incubated at 30°C for 3-7 days.

Transformants were analyzed by restriction enzyme digestion and Southern hybridization analysis. DNA was prepared from each of the candidates and digested with the same enzyme used to linearize the targeting plasmid. The Southern blot was probed with ³²P radiolabeled ARG4 DNA. Homologous integration events are identified by hybridization to a single band of exactly the same size as the linearized transforming DNA molecule [the "Unit Length Linear" band (ULL); Figure 2]. A ULL can only be generated if integration occurs into a DNA sequence that contains the restriction enzyme site in question, and contains enough homology surrounding that site to allow the re-synthesis (by repair) of the restriction enzyme site on the targeting plasmid. Candidates that display a ULL are assumed to be homologous integration events and are subjected to further analysis. Unit length linears were seen for 6 of 21 epsilon-globin candidates analyzed and for 3 of 14 beta-globin candidates. No unit length linears were observed for the other targeting sequences used.

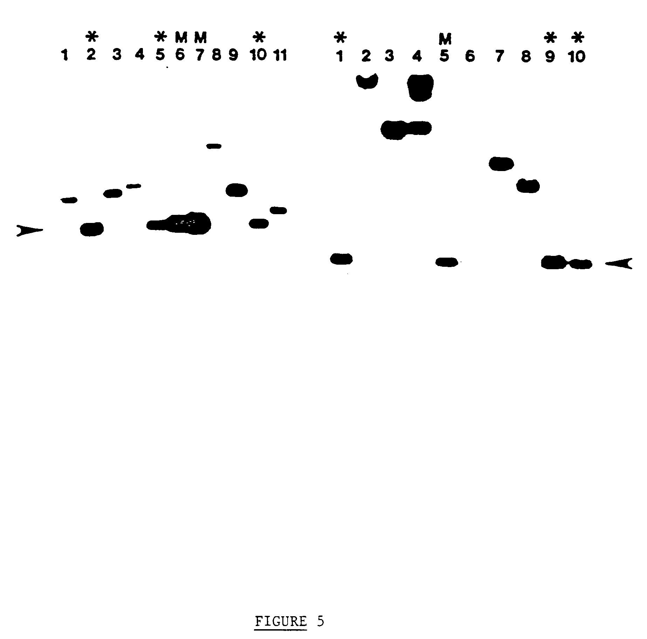

Figure 5 is a restriction enzyme and Southern blot analysis of clones selected by targeting with human epsilon- and beta-globin sequences. In the left panel, DNA from nine clones selected as arg+ were digested with AvaII (the enzyme used to make the double-strand break in the beta-globin targeting sequence). In the right panel, DNA from nine clones selected as arg+ were digested with ApaI (the enzyme used to make the double-strand break in the epsilon-globin targeting sequence). The asterisks identify clones correctly selected by homologous recombination. The lanes marked M were loaded with purified beta-globin targeting plasmid digested with AvaII (left panel), or purified epsilon-globin targeting plasmid digested with ApaI (right panel). The size of this marker fragment is identical to the size predicted for correctly targeted events. The arrowheads indicate the fragment size predicted for correctly targeted events, 5.6 kb in the left panel and 6.2 kb in the right panel. Hybridization was with ³²P labeled ARG4 DNA.

Three each of the beta- and epsilon-globin positives were further analyzed by CHEF gel electrophoresis (Chu, G., Vollrath, D., and David, R.W. Science, 234:1582-1585 (1986)), and restriction enzyme and Southern hybridization analysis, probing with epsilon- or beta-globin DNA as appropriate. This analysis demonstrated that all six YACs are identical and carry both beta- and epsilon-globin DNA, as would be expected since these two genes lie only 40 kb apart on human chromosome 11. In all six YACs the ARG4 DNA has integrated onto a YAC of 190 kb and the p184DLARG constructs have integrated as predicted into the homologous DNA within the globin locus.

Homologous recombination has been successfully used to isolate unique genes from a DNA YAC library. The YACs isolated encompass the entire beta-globin locus from at least 16 kb 5' to the epsilon gene down to the beta globin gene, along with about 130 kb of flanking DNA. In addition, a similar selection protocol performed with the same DNA YAC library resulted in the isolation of YACs from the β-globin locus after the library had been stored at -70°C for over fourteen months. It is thus disclosed here, for the first time, that it is possible to isolate clones from a human DNA YAC library by homologous-recombination selection.

### Saccharomyces Cerevisiae Host Strain Construction

The construction of strains of S. cerevisiae carrying chromosomal deletions of ARG4 (MGD131-10c) or ARG4 and TRP1 (IV-16d) can be carried out as follows:

### Deletion of ARG4:

The internal 2.0 kb HpaI fragment carrying the entire structural gene and regulatory elements for the yeast argininosuccinate lyase gene (ARG4) is deleted from a plasmid consisting of the 11 kb BamHI fragment isolated from p(SP013)2 (Wang, H-T., et al.,

Molecular and Cellular Biology, 7:1425-1435, 1987) inserted into the BamHI site of pUC19 (ATCC #37254), by digestion with HpaI and relegation of the DNA under dilute conditions (1 µg/ml). The resulting plasmid is digested with BamHI and introduced into an S. cerevisiae strain carrying the wild-type alleles for ARG4, TRP1, URA3, and LEU2, and carrying any non-reverting his3⁻ allele. The transformation is carried out in conjunction with any plasmid carrying yeast CEN and ARS elements, and the yeast HIS3 gene, using standard co-transformation conditions (Ausubel et al., 1989, Chapter 13). A useful plasmid for this purpose can readily be constructed by subcloning the 1.7 kb BamHI fragment from pRB15 (ATCC #37062) into the BamHI site of YCp50 (ATCC #37419). His⁺ Cells are screened for arginine auxotrophy by replica plating onto CM -arginine plates. His⁺ arg⁻ cells are grown in the absence of selection for HIS3, and single colonies are isolated and screened for histidine auxotrophs. DNA from his⁻ arg⁻ colonies is prepared and analyzed by restriction enzyme and Southern blot analysis to identify transformants carrying the ARG4 deletion (arg4Δ). This protocol is used to generate strain MGD131-10c used in Example I above.

### Deletion of TPP1:

In a yeast strain of opposite mating type as that used above, also carrying mutant alleles for LEU2 and URA3 (leu2⁻, ura2⁻), an identical procedure is carried out, but using a linear fragment of DNA carrying a deletion of the yeast gene for N-(5'-phosphoribosyl)-anthranilate isomerase (TRP1). This is accomplished by subcloning the BamHI-XhoI fragment from pBR322-Sc4120 (Stinchcomb, D.T., et al., Journal of Molecular Biology, 158:157-179, 1982) into BamHI-XhoI cut pGEM7, (Promega, Madison, Wisconsin) followed by deletion of the 1.2 kb EcoRi fragment containing TRP1 and ARS1. The resulting plasmid, pK2, is digested with BamHI and XhoI and co-transformed with a HIS3-CEN-ARS plasmid, like that described above, selecting for histidine prototrophs, and following the strategy outlined above to identify cells carrying the TRP1 deletion (trp1Δ). These cells are mated with cells carrying arg4Δ, and diploids heterozygous for the two deletions are isolated. This strain, TD7-16d (ATCC Accession Number 74010; genotype: a/α, arg4Δ/ARG4, LEU2/leu2-3,112, ura3-52/URA3, trp1- 289/trp1Δ, ade2-101/ade2-101, cyh^{S}/cyh^{r}, (CYH2/cyh2), his3Δ1/his3Δ1), is sporulated, subjected to tetrad analysis, and spores with appropriate phenotypes are analyzed by restriction enzyme and Southern blot analysis to identify a strain with both the arg4Δ and trp1Δ alleles (e.g., the haploid strain IV-16d).

### EXAMPLE II: SELECTION BY HOMOLOGOUS-RECOMBINATION OF A DNA CLONE FROM A DNA YAC LIBRARY USING ONE-STEP GENE DISRUPTION

The method of one-step gene disruption (Rothstein, R.J., Methods in Enzymology, 101:202-211, Academic Press, New York, 1983) can be adapted for use in the selection of clones from DNA libraries by homologous recombination. In this embodiment, a selectable marker is inserted into the targeting sequence. The targeting sequence, with the embedded selectable marker, is subsequently isolated as a single linear fragment (as diagrammed in Figure 3) and transformed into the pooled DNA YAC library, as described in Example I. Correctly targeted clones arising as a result of homologous recombination between the targeting molecule and specific DNA clones within the library will carry a single copy of the targeting sequence that is disrupted by the presence of the selectable marker, and will migrate at a specific and predictable position after restriction enzyme digestion and Southern blot analysis, using either ARG4 or the targeting sequence as a radiolabeled probe. This is in contrast to the process described in Example I, in which the correctly targeted DNA clones have two uninterrupted copies of the targeting sequence flanking the selectable marker.

Figure 3 illustrates the selection by homologous recombination of a DNA clone from a DNA YAC library using one-step gene disruption. The thin line represents an insert of DNA in the form of a yeast artificial chromosome (YAC). The solid box is the DNA fragment, a sequence of DNA constituting a portion of a DNA YAC clone found in the library that is homologous to the targeting sequence. In the diagram, the targeting sequence (solid boxes) has been modified by the insertion of the yeast ARG4 gene (open box). The remaining portions of the DNA YAC are comprised of the YAC vector arms: the thick lines represent plasmid sequences for replication and selection in bacteria. The shaded boxes represent genetic markers used for selection in yeast (yeast selectable markers URA3 and TRP1). The solid arrowheads and circle represent telomeres (TEL) and a centromere/ yeast replication origin (CEN/ARS), respectively. Figure 3a depicts the targeting molecule aligning with the target sequence on the DNA YAC. Figure 3b depicts the product of homologous recombination between the targeting and target sequences, with the targeting sequence having replaced the target sequence.

As a specific example of this embodiment of the basic concept, the 1.9 kb 5' epsilon-globin fragment (see Example I) is subcloned into the HindIII site of pUC18 (ATCC #37253). The resulting plasmid is digested with ApaI, dropping out a 0.26 kb ApaI fragment from the central portion of the 5' epsilon-globin insert. The 3' ApaI overhangs are made blunt with T4 DNA polymerase, and the resulting material is ligated to the purified ARG4 2.0 kb HpaI fragment (Beacham, I.R., Gene, 29:271-179, 1984). The resulting plasmid, with ARG4 disrupting the 5'epsilon-globin sequence, is digested with HindIII and transformed into the DNA YAC library, as described in Example I. The specific example presented results in the replacement of 0.26 kb of the 5' epsilon-globin DNA with the ARG4 sequence, since ApaI is not unique in the targeting sequence. For enzymes that are unique in the targeting sequence, however, the result will be a simple insertion.

### EXAMPLE III: USE OF TERMINAL FRAGMENTS DERIVED FROM YEAST ARTIFICIAL CHROMOSOME CLONES FOR THE ISOLATION OF CLONES KNOWN TO BE PRESENT IN A YEAST ARTIFICIAL CHROMOSOME LIBRARY--A MODEL SYSTEM TO TEST THE FEASIBILITY OF LIBRARY SCREENING BY HOMOLOGOUS RECOMBINATION

We used homologous recombination screening to extract a clone from the library that was known to exist within the library. Since the vector arm containing the TRP1 gene in YACs constructed with pYAC4 contains a plasmid replicon and a selectable marker (the beta-lactamase gene conferring ampicillin resistance), the technique of "plasmid rescue" was used to isolate terminal fragments from two YACs constructed in the vector pYAC4. The restriction enzyme XhoI cleaves at a single site within the TRP1 vector arm, at the junction between the telomere and pBR322 sequences. Complete digestion of YAC DNA with XhoI should produce a restriction fragment devoid of telomeric sequences, containing a functional plasmid replicon and Amp^{r} marker, and harboring a segment of human DNA that was adjacent to the vector arm in the original YAC clone and extends to the terminal XhoI site in the human DNA insert.

A group of 161 YACs within the library were constructed using the host yeast strain MGD131-10c (genotype a leu2-3,112 ADE2 cyh2^{r} hisΔ1 trp1-289 agr4Δ ura3-52). Total DNA from two clones in this group was digested with XhoI, ligated under dilute conditions to promote intramolecular circularization, and transformed into E. coli (all steps carried out essentially as described in Ausubel et al., 1988 [above]. Plasmid DNA was isolated from ampicillin resistant colonies and subjected to restriction enzyme analysis. One human DNA fragment from each of the two rescued plasmids was subsequently blunt-ended by treatment with T4 polymerase and ligated into the SmaI site of p184DLARG. The fragments, 10B and 8A, are 1 and 4 kb fragments, respectively, of human DNA lying adjacent to the TRP1 vector arms in two different YACs. The resulting constructs (plasmids p184-10B and p184-8A) were digested with a number of restriction enzymes which do not cleave p184DLARG to identify an enzyme that would cut within the human DNA to promote targeting. 20 µg of each construct was digested with the appropriate targeting enzyme and used for library screening, essentially as described in Example 1. Fragment 8A contains a single KpnI site lying 2.8 kb from one end and this enzyme was used to introduce a unique double strand break within the inserted sequence in p184-8A. Fragment 10B contains a single AvaII site lying 0.5 kb from one end and this enzyme was used to introduce a unique double strand break within the inserted sequence in p184-10B.

Eleven arg⁺ colonies resulting from screening with clone 8A were isolated and analyzed. Similar to strain IV-16d (Example 1), strain MGD131-10c carries a 2 kb deletion encompassing the entire ARG4 gene. However, the two strains differ with regard to their LEU2 genotype; IV-16d is leu⁺ and MGD131-10c has a leu⁻ phenotype. Seven of the 11 colonies displayed a leu⁻ phenotype, suggesting that they indeed represented independent isolates of the original YAC from which clone 8A was derived (a very strong possibility since strain MGD131-10c is the host for only 161 out of the 11,625 YACs (1.4%) in the library). Seventeen arg⁺ colonies resulting from screening with clone 10B were isolated and analyzed. Three of the 17 colonies displayed the leu⁻ phenotype. The presence of the leu⁻ marker strongly suggests that these clones represent isolates of the original YAC from which clone 10B was derived.

DNA was prepared from each of the seven leu⁻ colonies isolated by screening with clone 8A as well as one of the leu⁺ colonies. DNA was digested with the same enzyme used to linearize the transforming DNA molecule (KpnI). A Southern blot of these digests were probed with 32-P labeled ARG4 DNA. As described in Example 1, homologous integration events should reveal hybridization to a single fragment of exactly the same size as the linearized transforming DNA molecule (referred to in Example 1 as a Unit Length Linear Fragment, or ULL). Of the eight clones analyzed, all seven in strain MGD131-10c (the leu⁻ colonies) represent homologous events, while the single leu⁺ transformant analyzed does not (Figure 6). Thus, seven out of eleven candidate clones isolated were correctly targeted events. A similar analysis was performed on each of the three leu⁻ colonies isolated by screening with clone 10B. All three clones displayed a ULL upon Southern blot analysis, while 14 leu⁺ transformants did not.

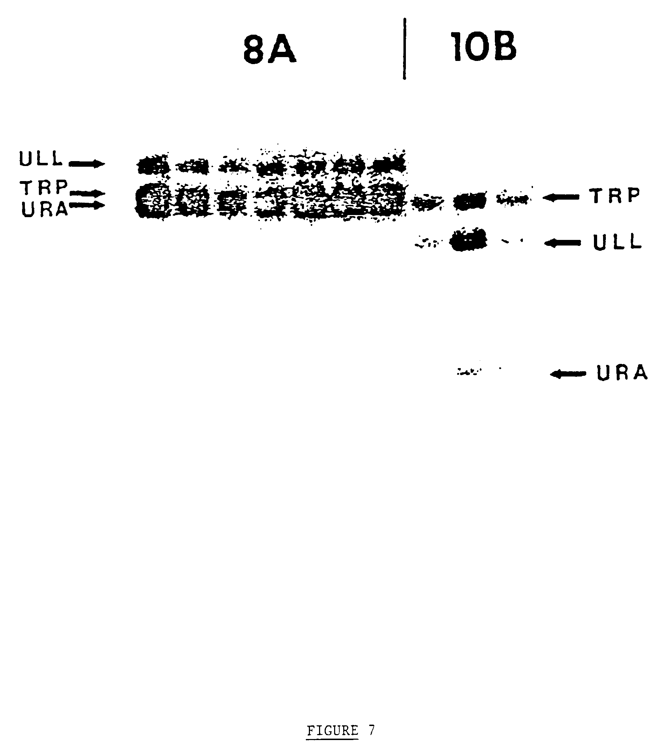

To confirm that the three homologous events isolated by screening with clone 10B and the seven homologous events isolated by screening with clone 8A represent the independent isolates of the same YACs, we have mapped the termini of the YACs in these ten clones. Figure 7 shows the result of this analysis. Three bands are evident in each lane, corresponding to the ULL, the left arm, and the right arm of the YAC. The bands migrate at identical positions in all seven YACs isolated with 8A, and at different, but identical positions in all three YACs isolated with 10B. These data show that the distance to the nearest KpnI site at each end of the seven 8A YACs is identical, while the three 10B YACs display similar behavior for the positions of their terminal AvaII sites.

### EXAMPLE IV: SCREENING OF A HUMAN YEAST ARTIFICIAL CHROMOSOME LIBRARY BY HOMOLOGOUS RECOMBINATION TO ISOLATE A YEAST ARTIFICIAL CHROMOSOME CLONE DERIVED FROM THE HUMAN ADENOSINE DEAMINASE LOCUS

Synthetic oligonucleotides o6 and o7-2 were used in the polymerase chain reaction to amplify a 1,376 base pair fragment of the human ADA gene corresponding to positions 34,243-35,618 (Genbank entry HUMADAG) from total human genomic DNA isolated from peripheral blood leukocytes. The amplified fragment was digested with PstI and the 852 base pair subfragment corresponding to HUMADAG positions 34,349-35,201 was isolated and cloned into the PstI site of plasmid p184DLARG (Example 1) to genetic p184DLARG/PCRF.5. One insert orientation was chosen (that with HUMADAG position 34,349 adjacent to the 3' end of the yeast ARG4 gene in p184DLARG. The resulting plasmid was purified and 20 micrograms was linearized at the unique EcoNI site within the human ADA insert (corresponding to HUMADAG position 34,657) prior to transformation into the pooled YAC library. Transformation of the pooled YAC library was performed exactly as described in Example 1, with the exception being that the YAC library consisted of an additional 3,585 clones, for a total of 15,210 clones representing approximately 1.2 genome equivalents.

Four arg⁺ transformants were isolated. Three of these are displayed in Figure 8 and all three displayed a unit-length linear fragment upon restriction enzyme digestion with EcoNI and Southern blot analysis. Analysis of the fourth arg⁺ transformant confirmed that it carries the same insert as YAC 184ADA.C and 184ADA.D. All four transformants harbor a similarly sized YAC of ca. 200 kb as judged by CHEF gel electrophoresis. The intensity of the ULL band in DNA prepared from YAC 184ADA.B and other data indicate that YAC 184ADA.B has undergone multiple tandem integrations of the targeting plasmid.

Comparison of a representative YAC, YAC 184ADA.C, with human genomic DNA by restriction enzyme and Southern hybridization analysis using multiple probes and restriction digests confirmed that this YAC indeed contains sequences from the human ADA locus. 
OLIGONUCLEOTIDE o6

10 20

5' AGATCTGTTT GAGGCTGCTG TGAG
Bases numbered 1-24 corresponding to positions 34,243-34,266 in GENBANK Entry HUMADAG. 
OLIGONUCLEOTIDE o7-2

10 20
5' AGATCCGGCA ACTTGTAGTA CCCAGGATG
Bases numbered 7-29 corresponding to positions 35,618-35,596 in GENBANK Entry HUMADAG. Bases 1-6 correspond to one of the four possible recognition sequences for the restriction enzyme BstYI, added to facilitate cloning.

### EXAMPLE V: QUANTIFICATION OF EFFECT OF CHROMOSOMAL DELETIONS OF HOMOLOGOUS SEQUENCES PRESENT IN HOST CELL

Orr-Weaver et al. (Proc. Natl. Acad. Sci. USA, Vol. 78, 10:6354-6358 (1981)) showed that a plasmid carrying the yeast LEU2 gene results in leu⁺ transformants at a frequency of 1.4-1.7 per µg of DNA when a double-strand break was made in the pBR322 portion of the plasmid. This is 1/10 of the frequency at which leu⁺ transformants arose when targeting was directed to the LEU2 gene by a double-strand break in LEU2 sequences (12-17 per µg of DNA). Similarly, when a HIS3 containing plasmid was cut within pBR322 sequences, his⁺ transformants appeared at 1/60 of the rate observed when the same plasmid was cut within HIS3. In both cases, the non-targeted prototrophs were demonstrated to be the results of recombination between the plasmid and the chromosomal leu2⁻ and his3⁻ mutant genes. Thus, screening a library for one clone out of 50,000 by homologous recombination without deletion of the chromosomal LEU2 gene would be expected to yield 5,000 leu⁺ transformants which arise through homologous recombination with the yeast genome when the targeting plasmid carries LEU2, even if a double-strand targeting break is made in another part of the plasmid. The results suggest, however, that deleting the chromosomal copies of LEU2 and HIS3 would eliminate virtually all of the nontargeted events.

The advantage of chromosomal deletions from host cells for the purposes of the method was quantified as follows: A plasmid carrying the yeast ARG4 ("target") and URA3 ("marker") genes was transformed into a mixture of yeast cells after making a double-strand break at the unique BclI site in the ARG4 sequence. All of the cells in the mixture had homology to URA3, but only 1 in 1,000 or 1 in 10,000 had homology to ARG4. This type of dilution experiment measures the relative frequencies of targeted and non-targeted events. For example, using 1 µg of DNA and a 1 to 1,000 dilution, the isolation of 5 yeast colonies by homologous recombination at ARG4 indicates that 5,000 cells were theoretically capable of a targeted event, but only 5/5,000 cells actually had the necessary homology at ARG4. The targeting frequency is therefore equivalent to 5,000 targeted events per µg in an undiluted culture. If, in the same experiment, 5 colonies were isolated that were independent of homology at ARG4 (recombination at URA3 or elsewhere, non-targeted events), the frequency of these non-targeted events is 5 per µg, and the ratio of targeted to non-targeted events in this experiment would be 1,000 to 1.

For the 1 in 1,000 dilution, 78 targeted transformants were isolated (by recombination with ARG4; equivalent to 78,000 targeted events) and 17 by recombination elsewhere (non-targeted events). At a dilution of 1 in 10,000, four targeted events (equivalent to 40,000 targeted events) and seven non-targeted events were isolated. The ratio of targeted to non-targeted events is thus (78,000 + 40,000) divided by (17 + 7), or 4,917 to 1. This ratio would lead to approximately 10 incorrect events for every one correct event when screening a library for a sequence present on 1 in 50,000 YACs, which is several-fold too high to be generally acceptable, although the use of URA3 as a targeting marker is clearly preferred over the use of the LEU2 or HIS3 markers previously used in targeting studies (Orr-Weaver et al., 1981). 84% (16 of 19 analyzed) of the non-targeted events where in fact due to recombination between the URA3 marker on the plasmid and the chromosomal ura3⁻ locus. If there were no homology between the targeting plasmid and the chromosomal ura3⁻ locus, then the non-targeted events resulting from homology at the ura3⁻ locus are removed from the analysis and the ratio increases to 30,729 to 1. At this ratio, a sequence represented 3 times in 50,000 YACs would be correctly targeted 1.8 times for every one non-targeted event. This ratio would also result in the favorable ratio of one correct event for every 1.6 incorrect events when screening a library for a sequence present on only 1 in 50,000 YACs.

These results indicate that the selection of a targeted clone from a DNA YAC library is feasible and particularly efficient in host yeast cells that carry no homology with selectable markers present on targeting vectors.

## Claims (Claims for the following Contracting State(s): AT, BE, CH, LI, DE, DK, FR, GB, GR, IT, LU, NL, SE)

1. A homologous-recombination method for identifying and isolating a target DNA fragment from a DNA fragment library constructed in eukaryotic host cells, comprising the steps of:
a) providing the DNA fragment library of introduced DNA in a population of eukaryotic host cells in which genetic recombination between DNA introduced into the eukaryotic host cells and DNA present in the eukaryotic host cells occurs by homologous recombination;
b) incorporating a selectable marker gene and a targeting DNA sequence linked to one another, into the population of the host eukaryotic cells containing the DNA fragment library, thereby producing a mixed population of eukaryotic host cells, the selectable marker gene being for selection in the host cell and the targeting DNA sequence being homologous in part to the target DNA fragment of the introduced DNA,
c) maintaining the mixed population of eukaryotic host cells under conditions appropriate for homologous recombination to occur, whereby only eukaryotic host cells containing the target DNA fragment of the introduced DNA are stably transformed with the selectable marker gene and the targeting DNA sequence as a result of homologous recombination between the target DNA fragment and the targeting DNA sequence and stably transformed eukaryotic host cells with a selectable phenotype are produced; and
d) culturing the product of step (c) under conditions appropriate for selection of stably transformed eukaryotic host cells with a selectable phenotype.

2. A method of Claim 1 wherein (i) the eukaryotic host cells are yeast host cells, the targeting DNA is present in a replicating linear vector contained within yeast cells which are of mating type opposite to the mating type of the yeast host cells and referred to as yeast cells containing the targeting DNA and the targeting DNA is introduced into yeast host cells by mating yeast host cells with yeast cells containing the targeting DNA; or
(ii) the targeting DNA vector is a linear DNA fragment, wherein the targeting DNA sequence is disrupted by the insertion therein of the selectable marker gene for selection of the eukaryotic host cell.

3. A homologous-recombination method for identifying and isolating a target DNA fragment from a DNA fragment library constructed in eukaryotic host cells, comprising the steps of:
a) providing a DNA fragment library in a population of eukaryotic host cells in which genetic recombination between DNA introduced into the eukaryotic host cells and DNA present in the eukaryotic host cells occurs by homologous recombination;
b) introducing a selectable marker gene and a targeting DNA sequence linked to one another, into the population of the host eukaryotic cells containing the DNA fragment library, thereby producing a mixed population of eukaryotic host cells, the selectable marker gene for selection in the host cell and the targeting DNA sequence homologous in part to a target DNA fragment of the introduced DNA,
c) maintaining the mixed population of eukaryotic host cells under conditions appropriate for homologous recombination to occur, whereby eukaryotic host cells containing the target DNA fragment are stably transformed with the selectable marker gene and the targeting DNA sequence present in the targeting DNA vector as a result of homologous recombination between the target DNA fragment and the targeting DNA sequence and stably transformed eukaryotic host cells with a selectable phenotype are produced; and
d) culturing the product of step (c) under conditions appropriate for selection of stably transformed eukaryotic host cells with a selectable phenotype.

4. A method of Claim 3 wherein (i) the targeting DNA molecule is a targeting plasmid, having for example a double-strand break introduced within the targeting DNA sequence; or
(ii) the target DNA fragment is cDNA.

5. A method of Claim 4 wherein (A) the targeting plasmid is a YIp plasmid and DNA fragments are present in eukaryotic host cells in artificial chromosomes, the artificial chromosomes additionally comprising all of the DNA sequences necessary for the chromosome to participate in host cell replication and chromosome segregation; or
(B) the target DNA fragment is: a mammalian DNA sequence which is an intergenic DNA sequence; a human DNA sequence which is an intergenic DNA sequence; a plant DNA sequence which is an intergenic DNA sequence; a mammalian gene or portion thereof; a human gene or portion thereof; or a plant gene or portion thereof.

6. A method of Claim 5 wherein the selectable marker gene is selected from the group consisting of genes which confer a selectable phenotype on eukaryotic host cells and the selectable phenotype is: antibiotic resistance, nutrient prototrophy, tolerance to a metal ion, ability to progress through the cell cycle or expression of a cell surface marker.

7. A homologous-recombination method for identifying and isolating a target DNA fragment in a DNA fragment library constructed in yeast cells, comprising the steps of:
a) providing a DNA fragment library in a population of host yeast cells, wherein DNA fragments are present in yeast artificial chromosomes and a host yeast cell contains one yeast artificial chromosome or a low-copy number of yeast artificial chromosomes;
b) introducing into the yeast cells containing the DNA fragment library a selectable marker gene for selection in yeast linked to targeting DNA homologous in part to a target DNA fragment, thereby producing a mixed population of yeast cells;
c) maintaining the mixed population of yeast cells under conditions appropriate for homologous recombination between targeting DNA and target DNA fragments present in yeast artificial chromosomes, whereby yeast cells containing the target DNA fragment are stably transformed with the selectable marker gene and the targeting DNA sequence as a result of homologous recombination between the target DNA fragment and the targeting DNA sequence and stably transformed yeast cells with a selectable phenotype are produced; and
d) culturing the product of step (c) under conditions appropriate for selection of yeast cells with a selectable phenotype.

8. A method of Claim 7 wherein the targeting DNA molecule is a linear DNA fragment, wherein the targeting DNA sequence is disrupted by the insertion of the selectable marker for yeast into the targeting DNA.

9. A homologous-recombination method for identifying and isolating a target DNA fragment in a DNA fragment library constructed in yeast cells, comprising the steps of:
a) providing a DNA fragment library in a population of host yeast cells, wherein DNA fragments are present in yeast artificial chromosomes and a host yeast cell contains one yeast artificial chromosome or a low-copy number of yeast artificial chromosomes;
b) introducing into the yeast cells containing the DNA fragment library a targeting DNA vector which is non-replicating in yeast, the targeting DNA vector having a selectable marker gene for selection in yeast and targeting DNA homologous in part to a target DNA fragment, thereby producing a mixed population of yeast cells;
c) maintaining the mixed population of yeast cells under conditions appropriate for homologous recombination between targeting DNA and target DNA fragments present in yeast artificial chromosomes, whereby yeast cells containing the target DNA fragment are stably transformed with the selectable marker gene and the targeting DNA sequence present in the targeting DNA vector as a result of homologous recombination between the target DNA fragment and the targeting DNA sequence and stably transformed yeast cells with a selectable phenotype are produced; and
d) culturing the product of step (c) under conditions appropriate for selection of yeast cells with a selectable phenotype.

10. A method of Claim 9 wherein the targeting DNA vector is a plasmid and the selectable phenotype is: antibiotic resistance, nutrient prototrophy, tolerance to a metal ion, ability to progress through the cell cycle, or expression of a cell surface marker; and for example wherein the targeting plasmid has a double-strand break introduced within the targeting DNA.

11. A homologous-recombination method for identifying and isolating a target DNA fragment in a yeast artificial chromosome library, comprising the steps of:
a) providing a yeast artificial chromosome library in a population of host yeast cells, wherein DNA fragments are present in the yeast artificial chromosomes and one copy or a low-copy number of the yeast artificial chromosomes is present in host yeast cells;
b) introducing into the yeast artificial chromosome library of (a) a targeting plasmid which is a bacterial plasmid non-replicating in yeast, the targeting plasmid having a selectable marker gene for selection in yeast and targeting DNA in which there is a double strand break;
c) maintaining the product of step (b) under conditions appropriate for homologous recombination between targeting DNA and target DNA fragments present in yeast artificial chromosomes, whereby homologous recombination between a target DNA fragment and targeting DNA produces host yeast cells having a selectable phenotype; and
d) selecting host yeast cells having the selectable phenotype conferred in step (c).

12. A method of Claim 10 or Claim 11 wherein the target DNA fragment is: a mammalian DNA sequence which is an intergenic DNA sequence; a human DNA sequence which is an intergenic DNA sequence; a plant DNA sequence which is an intergenic DNA sequence; a mammalian gene or portion thereof; a human gene or portion thereof; or a plant gene or portion thereof.

13. A DNA target fragment, isolated by the method of Claim 6 or Claim 12, which is a mammalian DNA sequence which is an intergenic DNA sequence; a human DNA sequence which is an intergenic DNA sequence; a plant DNA sequence which is an intergenic DNA sequence; a mammalian gene or portion thereof; a human gene or portion thereof; or a plant gene or portion thereof.

14. A method of Claim 11 wherein the selectable phenotype is: antibiotic resistance, nutrient prototrophy, tolerance to a metal ion, ability to progress through the cell cycle or expression of a cell surface marker.

15. A method of Claim 9 further comprising isolating contiguous DNA segments from the DNA fragment library, comprising the steps of:
a) subcloning a terminus of a target DNA fragment obtained by the method of Claim 9 into a targeting DNA vector which is non-replicating in yeast, the targeting DNA vector having a selectable marker gene for selection in yeast, thereby producing a targeting DNA vector containing subcloned DNA which is the terminus of the previously isolated target DNA fragment;
b) introducing into the DNA fragment library provided the targeting vector produced in (a) as subsequent targeting DNA;
c) maintaining the product of (b) under conditions appropriate for homologous recombination between targeting DNA and target DNA fragments in the DNA fragment library, thereby producing yeast cells which are stably transformed with the selectable marker gene and targeting DNA as a result of homologous recombination between the target DNA fragment and the targeting DNA sequence and have a selectable phenotype;
d) culturing the product of step (c) under conditions appropriate for selection of yeast cells in which homologous recombination has occurred;
e) selecting yeast cells, produced in (d), having a selectable phenotype;
f) subcloning a terminus of the target DNA fragment present in yeast cells selected in (e) into a targeting DNA vector as in step (a); and
g) repeating steps (b) through (e) as needed; and
for example wherein the targeting DNA vector is a bacterial plasmid having a double strand break in the targeting DNA therein and the selectable phenotype is selected from the group consisting of:
antibiotic resistance, nutrient prototrophy, tolerance to a metal ion and complementation of impaired function of a gene essential for progression through the cell cycle.

16. Saccharomyces cerevisiae carrying a chromosomal deletion of the complete coding region of two or more selectable marker genes which confer for example nutrient prototrophy.

17. Any of Saccharomyces cerevisiae TD7-16d(ATCC 74010);
Saccharomyces cerevisiae IV-16d; and
Saccharomyces cerevisiae MGD131-10c.

18. Plasmid p184DLARG and functional equivalents thereof.

19. A plasmid non-replicating in yeast, comprising:
a) a yeast selectable marker gene;
b) a bacterial origin of replication;
c) a bacterial selectable marker gene; and
d) a cloning site for insertion of targeting DNA.

20. Saccharomyces cerevisiae carrying a chromosomal deletion of the argininosuccinate lyase gene and a chromosomal deletion of the N-(5'-phosphoribosyl)-anthranilate isomerase gene.

## Claims (Claims for the following Contracting State(s): ES)

1. A homologous-recombination method for identifying and isolating a target DNA fragment from a DNA fragment library constructed in eukaryotic host cells, comprising the steps of:
a) providing the DNA fragment library of introduced DNA in a population of eukaryotic host cells in which genetic recombination between DNA introduced into the eukaryotic host cells and DNA present in the eukaryotic host cells occurs by homologous recombination;
b) incorporating a selectable marker gene and a targeting DNA sequence linked to one another, into the population of the host eukaryotic cells containing the DNA fragment library, thereby producing a mixed population of eukaryotic host cells, the selectable marker gene being for selection in the host cell and the targeting DNA sequence being homologous in part to the target DNA fragment of the introduced DNA,
c) maintaining the mixed population of eukaryotic host cells under conditions appropriate for homologous recombination to occur, whereby only eukaryotic host cells containing the target DNA fragment of the introduced DNA are stably transformed with the selectable marker gene and the targeting DNA sequence as a result of homologous recombination between the target DNA fragment and the targeting DNA sequence and stably transformed eukaryotic host cells with a selectable phenotype are produced; and
d) culturing the product of step (c) under conditions appropriate for selection of stably transformed eukaryotic host cells with a selectable phenotype.

2. A method of Claim 1 wherein (i) the eukaryotic host cells are yeast host cells, the targeting DNA is present in a replicating linear vector contained within yeast cells which are of mating type opposite to the mating type of the yeast host cells and referred to as yeast cells containing the targeting DNA and the targeting DNA is introduced into yeast host cells by mating yeast host cells with yeast cells containing the targeting DNA; or (ii) the targeting DNA vector is a linear DNA fragment, wherein the targeting DNA sequence is disrupted by the insertion therein of the selectable marker gene for selection of the eukaryotic host cell.

3. A homologous-recombination method for identifying and isolating a target DNA fragment from a DNA fragment library constructed in eukaryotic host cells, comprising the steps of:
a) providing a DNA fragment library in a population of eukaryotic host cells in which genetic recombination between DNA introduced into the eukaryotic host cells and DNA present in the eukaryotic host cells occurs by homologous recombination;
b) introducing a selectable marker gene and a targeting DNA sequence linked to one another, into the population of the host eukaryotic cells containing the DNA fragment library, thereby producing a mixed population of eukaryotic host cells, the selectable marker gene for selection in the host cell and the targeting DNA sequence homologous in part to a target DNA fragment of the introduced DNA,
c) maintaining the mixed population of eukaryotic host cells under conditions appropriate for homologous recombination to occur, whereby eukaryotic host cells containing the target DNA fragment are stably transformed with the selectable marker gene and the targeting DNA sequence present in the targeting DNA vector as a result of homologous recombination between the target DNA fragment and the targeting DNA sequence and stably transformed eukaryotic host cells with a selectable phenotype are produced; and
d) culturing the product of step (c) under conditions appropriate for selection of stably transformed eukaryotic host cells with a selectable phenotype.

4. A method of Claim 3 wherein (i) the targeting DNA molecule is a targeting plasmid, having for example a double-strand break introduced within the targeting DNA sequence, or
(ii) the target DNA fragment is cDNA.

5. A method of claim 4 wherein (A) the targeting plasmid is a YIp plasmid and DNA fragments are present in eukaryotic host cells in artificial chromosomes, the artificial chromosomes additionally comprising all of the DNA sequences necessary for the chromosome to participate in host cell replication and chromosome segregation; or
(B) the target DNA fragment is: a mammalian DNA sequence which is an intergenic DNA sequence; a human DNA sequence which is an intergenic DNA sequence; a plant DNA sequence which is an intergenic DNA sequence; a mammalian gene or portion thereof; a human gene or portion thereof; or a plant gene or portion thereof.

6. A method of Claim 5 wherein the selectable marker gene is selected from the group consisting of genes which confer a selectable phenotype on eukaryotic host cells and the selectable phenotype is: antibiotic resistance, nutrient prototrophy, tolerance to a metal ion, ability to progress through the cell cycle or expression of a cell surface marker.

7. A homologous-recombination method for identifying and isolating a target DNA fragment in a DNA fragment library constructed in yeast cells, comprising the steps of:
a) providing a DNA fragment library in a population of host yeast cells, wherein DNA fragments are present in yeast artificial chromosomes and a host yeast cell contains one yeast artificial chromosome or a low-copy number of yeast artificial chromosomes;
b) introducing into the yeast cells containing the DNA fragment library a selectable marker gene for selection in yeast linked to targeting DNA homologous in part to a target DNA fragment, thereby producing a mixed population of yeast cells;
c) maintaining the mixed population of yeast cells under conditions appropriate for homologous recombination between targeting DNA and target DNA fragments present in yeast artificial chromosomes, whereby yeast cells containing the target DNA fragment are stably transformed with the selectable marker gene and the targeting DNA sequence as a result of homologous recombination between the target DNA fragment and the targeting DNA sequence and stably transformed yeast cells with a selectable phenotype are produced; and
d) culturing the product of step (c) under conditions appropriate for selection of yeast cells with a selectable phenotype.

8. A method of Claim 7 wherein the targeting DNA molecule is a linear DNA fragment, wherein the targeting DNA sequence is disrupted by the insertion of the selectable marker for yeast into the targeting DNA.

9. A homologous-recombination method for identifying and isolating a target DNA fragment in a DNA fragment library constructed in yeast cells, comprising the steps of:
a) providing a DNA fragment library in a population of host yeast cells, wherein DNA fragments are present in yeast artificial chromosomes and a host yeast cell contains one yeast artificial chromosome or a low-copy number of yeast artificial chromosomes;
b) introducing into the yeast cells containing the DNA fragment library a targeting DNA vector which is non-replicating in yeast, the targeting DNA vector having a selectable marker gene for selection in yeast and targeting DNA homologous in part to a target DNA fragment, thereby producing a mixed population of yeast cells;
c) maintaining the mixed population of yeast cells under conditions appropriate for homologous recombination between targeting DNA and target DNA fragments present in yeast artificial chromosomes, whereby yeast cells containing the target DNA fragment are stably transformed with the selectable marker gene and the targeting DNA sequence present in the targeting DNA vector as a result of homologous recombination between the target DNA fragment and the targeting DNA sequence and stably transformed yeast cells with a selectable phenotype are produced; and
d) culturing the product of step (c) under conditions appropriate for selection of yeast cells with a selectable phenotype.

10. A method of Claim 9 wherein the targeting DNA vector is a plasmid and the selectable phenotype is: antibiotic resistance, nutrient prototrophy, tolerance to a metal ion, ability to progress through the cell cycle, or expression of a cell surface marker; and for example wherein the targeting plasmid has a double-strand break introduced within the targeting DNA.

11. A homologous-recombination method for identifying and isolating a target DNA fragment in a yeast artificial chromosome library, comprising the steps of:
a) providing a yeast artificial chromosome library in a population of host yeast cells, wherein DNA fragments are present in the yeast artificial chromosomes and one copy or a low-copy number of the yeast artificial chromosomes is present in host yeast cells;
b) introducing into the yeast artificial chromosome library of (a) a targeting plasmid which is a bacterial plasmid non-replicating in yeast, the targeting plasmid having a selectable marker gene for selection in yeast and targeting DNA in which there is a double strand break;
c) maintaining the product of step (b) under conditions appropriate for homologous recombination between targeting DNA and target DNA fragments present in yeast artificial chromosomes, whereby homologous recombination between a target DNA fragment and targeting DNA produces host yeast cells having a selectable phenotype; and
d) selecting host yeast cells having the selectable phenotype conferred in step (c).

12. A method of Claim 10 or Claim 11 wherein the target DNA fragment is: a mammalian DNA sequence which is an intergenic DNA sequence; a human DNA sequence which is an intergenic DNA sequence; a plant DNA sequence which is an intergenic DNA sequence; a mammalian gene or portion thereof; a human gene or portion thereof; or a plant gene or portion thereof.

13. A method of isolating a DNA target fragment by the method of Claim 6 or Claim 12, wherein the target DNA fragment is: a mammalian DNA sequence which is an intergenic DNA sequence; a human DNA sequence which is an intergenic DNA sequence; a plant DNA sequence which is an intergenic DNA sequence; a mammalian gene or portion thereof; a human gene or portion thereof; or a plant gene or portion thereof.

14. A method of Claim 11 wherein the selectable phenotype is: antibiotic resistance, nutrient prototrophy, tolerance to a metal ion, ability to progress through the cell cycle or expression of a cell surface marker.

15. A method of Claim 9 further comprising isolating contiguous DNA segments from the DNA fragment library, comprising the steps of:
a) subcloning a terminus of a target DNA fragment obtained by the method of Claim 9 into a targeting DNA vector which is non-replicating in yeast, the targeting DNA vector having a selectable marker gene for selection in yeast, thereby producing a targeting DNA vector containing subcloned DNA which is the terminus of the previously isolated target DNA fragment;
b) introducing into the DNA fragment library provided the targeting vector produced in (a) as subsequent targeting DNA;
c) maintaining the product of (b) under conditions appropriate for homologous recombination between targeting DNA and target DNA fragments in the DNA fragment library, thereby producing yeast cells which are stably transformed with the selectable marker gene and targeting DNA as a result of homologous recombination between the target DNA fragment and the targeting DNA sequence and have a selectable phenotype;
d) culturing the product of step (c) under conditions appropriate for selection of yeast cells in which homologous recombination has occurred;
e) selecting yeast cells, produced in (d), having a selectable phenotype;
f) subcloning a terminus of the target DNA fragment present in yeast cells selected in (e) into a targeting DNA vector as in step (a); and
g) repeating steps (b) through (e) as needed; and
for example wherein the targeting DNA vector is a bacterial plasmid having a double strand break in the targeting DNA therein and the selectable phenotype is selected from the group consisting of:
antibiotic resistance, nutrient prototrophy, tolerance to a metal ion and complementation of impaired function of a gene essential for progression through the cell cycle.

16. Saccharomyces cerevisiae carrying a chromosomal deletion of the complete coding region of two or more selectable marker genes which confer for example nutrient prototrophy.

17. Any of Saccharomyces cerevisiae TD7-16d(ATCC 74010);
Saccharomyces cerevisiae IV-16d; and
Saccharomyces cerevisiae MGD131-10c.

18. A method of making a Plasmid p184DLARG and functional equivalents thereof including the step of providing the plasmid with yeast ARG4 gene as a selectable marker.

19. A method of making a plasmid non-replicating in yeast, comprising the steps of:
a) providing a yeast selectable marker gene;
b) providing a bacterial origin of replication;
c) providing a bacterial selectable marker gene; and
d) providing a cloning site for insertion of targeting DNA.

20. Saccharomyces cerevisiae carrying a chromosomal deletion of the argininosuccinate lyase gene and a chromosomal deletion of the N-(5'-phosphoribosyl)-anthranilate isomerase gene.

## Patentansprüche (Patentansprüche für folgende(n) Vertragsstaat(en): AT, BE, CH, LI, DE, DK, FR, GB, GR, IT, LU, NL, SE)

1. Ein homologes Rekombinationsverfahren zur Identifizierung und Isolierung eines Ziel-DNS-Fragments aus einer in eukaryontischen Wirtszellen konstruierten DNS-Fragmentbibliothek, wobei das Verfahren die Schritte umfaßt:
a) Bereitstellen der DNS-Fragmentbibliothek einer DNS, die in eine Population eukaryontischer Wirtszellen eingeführt ist, in denen eine genetische Rekombination zwischen in die eukaryontischen Wirtszellen eingeführter und in den eukaryontischen Wirtszellen vorhandener DNS durch homologe Rekombination stattfindet;
b) Einbau eines selektierbaren Markergens und einer zielenden DNS-Sequenz, die miteinander verbunden sind, in die Population der eukaryontischen Wirtszellen, die die DNS-Fragmentbibliothek enthalten, wodurch eine gemischte Population eukaryontischer Wirtszellen gebildet wird, wobei das selektierbare Markergen zur Selektion in der Wirtszelle ist, und die zielende DNS-Sequenz teilweise homolog zum Ziel-DNS-Fragment der eingeführten DNS ist,
c) Halten der gemischten Population eukaryontischer Wirtszellen unter geeigneten Bedingungen, um eine homologe Rekombination stattfinden zu lassen, wodurch nur eukaryontische Wirtszellen, die das Ziel-DNS-Fragment der eingeführten DNS enthalten, stabil mit dem selektierbaren Markergen und der zielenden DNS-Sequenz transformiert sind als ein Ergebnis der homologen Rekombination zwischen dem Ziel-DNS-Fragment und der zielenden DNS-Sequenz, und es werden stabil transformierte eukaryontische Wirtszellen mit einem selektierbaren Phänotyp gebildet; und
d) Kultivieren des Produkts von Schritt (c) unter geeigneten Bedingungen zur Selektion stabil transformierter eukaryontischer Wirtszellen mit einem selektierbaren Phänotyp.

2. Ein Verfahren gemäß Anspruch 1, worin (i) die eukaryontischen Wirtszellen Hefewirtszellen sind, die Zielende DNS in einem replizierenden linearen Vektor vorhanden ist, der in Hefezellen enthalten ist, die von einem gegensätzlichen Paarungstyp zum Paarungstyp der Hefewirtszellen sind und als Hefezellen bezeichnet sind, die die zielende DNS enthalten, und die zielende DNS in Hefewirtszellen durch Paarung von Hefewirtszellen mit Hefezellen, die die zielende DNS enthalten, eingeführt wird; oder
ii) der zielende DNS-Vektor ein lineares DNS-Fragment ist, wobei die zielende DNS-Sequenz durch die Insertion des selektierbaren Markergens zur Selektion der eukaryontischen Wirtszelle unterbrochen ist.

3. Ein homologes Rekombinationsverfahren zur Identifizierung und Isolierung eines Ziel-DNS-Fragments aus einer in eukaryontischen Wirtszellen konstruierten DNS-Fragmentbibliothek, wobei das Verfahren die Schritte umfaßt:
a) Bereitstellen einer DNS-Fragmentbibliothek in einer Population eukaryontischer Wirtszellen, in denen eine genetische Rekombination zwischen in die eukaryontischen Wirtszellen eingeführter DNS und in den eukaryontischen Wirtszellen vorhandener DNS durch homologe Rekombination stattfindet;
b) Einführen eines selektierbaren Markergens und einer zielenden DNS-Sequenz, die miteinander verbunden sind, in die Population der eukaryontischen Wirtszellen, die die DNS-Fragmentbibliothek enthalten, wodurch eine gemischte Population eukaryontischer Wirtszellen gebildet wird, wobei das selektierbare Markergen zur Selektion in der Wirtszelle ist, und die zielende DNS-Sequenz teilweise homolog zu einem Ziel-DNS-Fragment der eingeführten DNS ist,
c) Halten der gemischten Population eukaryontischer Wirtszellen unter geeigneten Bedingungen, um eine homologe Rekombination stattfinden zu lassen, wobei das Ziel-DNS-Fragment enthaltende eukaryontische Wirtszellen stabil mit dem selektierbaren Markergen und der in dem zielenden DNS-Vektor vorhandenen zielenden DNS-Sequenz transformiert sind als ein Ergebnis einer homologen Rekombination zwischen dem Ziel-DNS-Fragment und der zielenden DNS-Sequenz, und es werden stabil transformierte eukaryontische Wirtszellen mit einem selektierbaren Phänotyp gebildet; und
d) Kultivieren des Produkts von Schritt (c) unter geeigneten Bedingungen zur Selektion stabil transformierter eukaryontischer Wirtszellen mit einem selektierbaren Phänotyp.

4. Ein Verfahren gemäß Anspruch 3, worin (i) das zielende DNS-Molekül ein zielendes Plasmid mit beispielsweise einem in die zielende DNS-Sequenz eingeführten Doppelstrangbruch ist, oder
(ii) das Ziel-DNS-Fragment cDNS ist.

5. Ein Verfahren gemäß Anspruch 4, worin (A) das zielende Plasmid ein YIp Plasmid ist, und DNS-Fragmente in eukaryontischen Wirtszellen in künstlichen Chromosomen vorhanden sind, wobei die künstlichen Chromosomen zusätzlich sämtliche DNS-Sequenzen aufweisen, die für die Teilnahme der Chromosomen an der Wirtszellreplikation und Chromosomen-Segregation notwendig sind; oder (B)das Ziel-DNS-Fragment ist: eine Säuger DNS-Sequenz, die eine intergene DNS-Sequenz ist; eine menschliche DNS-Sequenz, die eine intergene DNS-Sequenz ist; eine pflanzliche DNS-Sequenz, die eine intergene DNS-Sequenz ist; ein Säuger-Gen oder ein Teil davon; ein menschliches Gen oder ein Teil davon; oder ein pflanzliches Gen oder ein Teil davon.

6. Ein Verfahren gemäß Anspruch 5, worin das selektierbare Markergen aus der Gruppe der Gene gewählt ist, die eukaryontischen Wirtszellen einen selektierbaren Phänotyp verleihen, und der selektierbare Phänotyp ist: Antibiotikumresistenz, nutrititive Prototrophie, Metallionentoleranz, Fähigkeit, den Zellzyklus zu durchlaufen oder Expression eines Zelloberflächenmarkers.

7. Ein homologes Rekombinationsverfahren zur Identifizierung und Isolierung eines Ziel-DNS-Fragments in einer in Hefezellen konstruierten DNS-Fragmentbibliothek, wobei das Verfahren die Schritte umfaßt:
a) Bereitstellen einer DNS-Fragmentbibliothek in einer Population Hefewirtszellen, worin DNS-Fragmente in künstlichen Hefechromosomen vorhanden sind, und eine Hefewirtszelle ein künstliches Hefechromosom oder eine low-copy-number künstlicher Hefechromosomen enthält;
b) Einführen eines selektierbaren Markergens zur Selektion in Hefe, das an eine zielende DNS gebunden ist, die teilweise homolog zu einem Ziel-DNS-Fragment ist, in die Hefezellen, die die DNS-Fragmentbibliothek enthalten, wodurch eine gemischte Population Hefezellen gebildet wird;
c) Halten der gemischten Hefezellpopulation unter geeigneten Bedingungen für eine homologe Rekombination zwischen zielender DNS und in den künstlichen Hefechromosomen vorhandenen Ziel-DNS-Fragmenten, wodurch Hefezellen, die das Ziel-DNS-Fragment enthalten, stabil mit dem selektierbaren Markergen und der zielenden DNS-Sequenz transformiert sind als ein Ergebnis der homologen Rekombination zwischen dem Ziel-DNS-Fragment und der zielenden DNS-Sequenz, und es werden stabil transformierte Hefezellen mit einem selektierbaren Phänotyp gebildet; und
d) Kultivieren des Produkts von Schritt (c) unter geeigneten Bedingungen zur Selektion von Hefezellen mit einem selektierbaren Phänotyp.

8. Ein Verfahren gemäß Anspruch 7, worin das zielende DNS-Molekül ein lineares DNS-Fragment ist, worin die zielende DNS-Sequenz durch die Insertion des selektierbaren Markers für Hefe in der zielenden DNS unterbrochen wird.

9. Ein homologes Rekombinationsverfahren zur Identifizierung und Isolierung eines Ziel-DNS-Fragments in einer in Hefezellen konstruierten DNS-Fragmentbibliothek, wobei das Verfahren die Schritte umfaßt:
a) Bereitstellen einer DNS-Fragmentbibliothek in einer Population aus Hefewirtszellen, worin DNS-Fragmente in künstlichen Hefechromosomen vorhanden sind, und eine Hefewirtszelle ein künstliches Hefechromosom oder eine low-copy-number künstlicher Hefechromosomen enthält;
b) Einführen eines in Hefe nicht-replizierenden zielenden DNS-Vektors in die Hefezellen, die die DNS-Fragmentbibliothek enthalten, wobei der zielende DNS-Vektor ein selektierbares Markergen zur Selektion in Hefe besitzt, und die zielende DNS teilweise zu einem Ziel-DNS-Fragment homolog ist, wodurch eine gemischte Hefezellpopulation gebildet wird;
c) Halten der gemischten Hefezellpopulation unter geeigneten Bedingungen für eine homologe Rekombination zwischen zielender DNS und in den künstlichen Hefechromosomen vorhandenen Ziel-DNS-Fragmenten, wodurch Hefezellen, die das Ziel-DNS-Fragment enthalten, stabil mit dem selektierbaren Markergen und der im zielenden DNS-Vektor enthaltenen zielenden DNS-Sequenz transformiert sind als ein Ergebnis der homologen Rekombination zwischen dem Ziel-DNS-Fragment und der zielenden DNS-Sequenz, und es werden stabil transformierte Hefezellen mit einem selektierbaren Phänotyp gebildet; und
d) Kultivieren des Produkts von Schritt (c) unter geeigneten Bedingungen zur Selektion von Hefezellen mit einem selektierbaren Phänotyp.

10. Ein Verfahren gemäß Anspruch 9, worin der zielende DNS-Vektor ein Plasmid ist und der selektierbare Phänotyp ist: Antibiotikumresistenz, nutrititive Prototrophie, Metallionentoleranz, Fähigkeit, den Zellzyklus zu durchlaufen oder Expression eines Zelloberflächenmarkers; und beispielsweise worin das zielende Plasmid einen in die zielende DNS eingeführten Doppelstrangbruch besitzt.

11. Ein homologes Rekombinationsverfahren zur Identifizierung und Isolierung eines Ziel-DNS-Fragments in einer künstlichen Hefechromosombibliothek, wobei das Verfahren die Schritte umfaßt:
a) Bereitstellen einer künstlichen Hefechromosombibliothek in einer Population Hefewirtszellen, worin DNS-Fragmente in künstlichen Hefechromosomen vorhanden sind und eine Einzelkopie oder eine low-copy-number künstlicher Hefechromosomen in Hefewirtszellen vorhanden ist;
b) Einführen eines zielenden Plasmids, das ein in Hefe nicht-replizierendes bakterielles Plasmid ist, in die künstliche Hefechromosombibliothek von (a), wobei das zielende Plasmid ein selektierbares Markergen zur Selektion in Hefe und eine zielende DNS mit einem Doppelstrangbruch besitzt;
c) Halten des Produkts von Schritt (b) unter geeigneten Bedingungen für eine homologe Rekombination zwischen der zielenden DNS und in den künstlichen Hefechromosomen vorhandenen Ziel-DNS-Fragmenten, wobei homologe Rekombination zwischen einem Ziel-DNS-Fragment und zielender DNS Hefewirtszellen bildet, die einen selektierbaren Phänotyp besitzen; und
d) Selektion der Hefewirtszellen, die den in Schritt (c) verliehenen selektierbaren Phänotyp besitzen.

12. Ein Verfahren gemäß Anspruch 10 oder Anspruch 11, worin das Ziel-DNS-Fragment ist: eine Säuger DNS-Sequenz, die eine intergene DNS-Sequenz ist; eine menschliche DNS-Sequenz, die eine intergene DNS-Sequenz ist; eine pflanzliche DNS-Sequenz, die eine intergene DNS-Sequenz ist; ein Säuger-Gen oder ein Teil davon; ein menschliches Gen oder ein Teil davon; oder ein pflanzliches Gen oder ein Teil davon.

13. Ein mit dem Verfahren gemäß Anspruch 6 oder Anspruch 12 isoliertes DNS-Ziel-Fragment, das eine Säuger DNS-Sequenz ist, die eine intergene DNS-Sequenz ist; eine menschliche DNS-Sequenz, die eine intergene DNS-Sequenz ist; eine pflanzliche DNS-Sequenz, die eine intergene DNS-Sequenz ist; ein Säuger-Gen oder ein Teil davon; ein menschliches Gen oder ein Teil davon; oder ein pflanzliches Gen oder ein Teil davon.

14. Ein Verfahren gemäß Anspruch 11, worin der selektierbare Phänotyp ist: Antibiotikumresistenz, nutrititive Prototrophie, Metallionentoleranz, Fähigkeit, den Zellzyklus zu durchlaufen oder Expression eines Zelloberflächenmarkers.

15. Ein Verfahren gemäß Anspruch 9, das des weiteren Isolieren angrenzender DNS-Segmente aus der DNS-Fragmentbibliothek umfaßt, wobei das Verfahren die Schritte umfaßt:
a) Subklonieren eines Ziel-DNS-Fragmentendes, das mit dem Verfahren gemäß Anspruch 9 erhalten wird, in einen zielenden DNS-Vektor, der in Hefe nicht-replizierend ist, wobei der zielende DNS-Vektor ein selektierbares Markergen zur Selektion in Hefe besitzt, wodurch ein zielender DNS-Vektor gebildet wird, der subklonierte DNS enthält, die das Ende des zuvor isolierten DNS-Fragments ist;
b) Einführen des zielenden Vektors, der in (a) als nachfolgende zielende DNS gebildet wird, in die bereitgestellte DNS-Fragmentbibliothek;
c) Halten des Produkts von (b) unter geeigneten Bedingungen für eine homologe Rekombination zwischen zielender DNS und Ziel-DNS-Fragmenten in der DNS-Fragmentbibliothek, wodurch Hefezellen gebildet werden, die stabil mit dem selektierbaren Markergen und zielender DNS transformiert sind als ein Ergebnis der homologen Rekombination zwischen dem Ziel-DNS-Fragment und der zielenden DNS-Sequenz und einen selektierbaren Phänotyp besitzen;
d) Kultivieren des Produkts von Schritt (c) unter geeigneten Bedingungen zur Selektion von Hefezellen, in denen eine homologe Rekombination stattgefunden hat;
e) Selektion der in Schritt (d) gebildeten Hefezellen, die einen selektierbaren Phänotyp besitzen;
f) Subklonieren eines Ziel-DNS-Fragmentendes, das in (e) selektierten Hefezellen vorhandenen ist, in einen zielenden DNS-Vektor wie in Schritt (a); und
g) Wiederholen der Schritte (b) bis (e) je nach Bedarf; und beispielsweise worin der zielende DNS-Vektor ein bakterielles Plasmid mit einem Doppelstrangbruch innerhalb der zielenden DNS ist, und der selektierbare Phänotyp ausgewählt ist aus der Gruppe enthaltend: Antibiotikumresistenz, nutrititive Prototrophie, Metallionentoleranz und Komplementation der unvollständigen Funktion eines Gens, das für das Durchlaufen des Zellzyklus essentiell ist.

16. Saccharomyces cerevisiae mit einer chromosomalen Deletion der gesamten codierenden Region zweier oder mehrerer selektierbarer Markergene, die beispielsweise nutrititive Prototrophie verleihen.

17. Ein
Saccharomyces cerevisiae TD7-16d (ATCC 74010);
Saccharomyces cerevisiae IV-16d; und
Saccharomyces cerevisiae MGD131-10c.

18. Plasmid p184DLARG und funktionelle Äquivalente davon.

19. Ein in Hefe nicht replizierendes Plasmid, das umfaßt:
a) Ein selektierbares Hefe-Markergen;
b) Einen bakteriellen Replikationsursprung;
c) Ein selektierbares Bakterien-Markergen; und
d) Eine Klonierungsstelle zur Insertion einer zielenden DNS.

20. Saccharomyces cerevisiae mit einer chromosomalen Deletion des Argininsuccinat-Lyase-Gens und einer chromosomalen Deletion des N-(5'-phosphoribosyl)-anthranilat-Isomerase-Gens.

## Patentansprüche (Patentansprüche für folgende(n) Vertragsstaat(en): ES)

1. Ein homologes Rekombinationsverfahren zur Identifizierung und Isolierung eines Ziel-DNS-Fragments aus einer in eukaryontischen Wirtszellen konstruierten DNS-Fragmentbibliothek, wobei das Verfahren die Schritte umfaßt:
a) Bereitstellen der DNS-Fragmentbibliothek einer DNS, die in eine Population eukaryontischer Wirtszellen eingeführt ist, in denen eine genetische Rekombination zwischen in die eukaryontischen Wirtszellen eingeführter und in den eukaryontischen Wirtszellen vorhandener DNS durch homologe Rekombination stattfindet;
b) Einbau eines selektierbaren Markergens und einer zielenden DNS-Sequenz, die miteinander verbunden sind, in die Population der eukaryontischen Wirtszellen, die die DNS-Fragmentbibliothek enthalten, wodurch eine gemischte Population eukaryontischer Wirtszellen gebildet wird, wobei das selektierbare Markergen zur Selektion in der Wirtszelle ist, und die zielende DNS-Sequenz teilweise homolog zum ZielDNS-Fragment der eingeführten DNS ist,
c) Halten der gemischten Population eukaryontischer Wirtszellen unter geeigneten Bedingungen, um eine homologe Rekombination stattfinden zu lassen, wodurch nur eukaryontische Wirtszellen, die das Ziel-DNS-Fragment der eingeführten DNS enthalten, stabil mit dem selektierbaren Markergen und der zielenden DNS-Sequenz transformiert sind als ein Ergebnis der homologen Rekombination zwischen dem Ziel-DNS-Fragment und der zielenden DNS-Sequenz, und es werden stabil transformierte eukaryontische Wirtszellen mit einem selektierbaren Phänotyp gebildet; und
d) Kultivieren des Produkts von Schritt (c) unter geeigneten Bedingungen zur Selektion stabil transformierter eukaryontischer Wirtszellen mit einem selektierbaren Phänotyp.

2. Ein Verfahren gemäß Anspruch 1, worin (i) die eukaryontischen Wirtszellen Hefewirtszellen sind, die zielende DNS in einem replizierenden linearen Vektor vorhanden ist, der in Hefezellen enthalten ist, die von einem gegensätzlichen Paarungstyp zum Paarungstyp der Hefewirtszellen sind und als Hefezellen bezeichnet sind, die die zielende DNS enthalten, und die zielende DNS in Hefewirtszellen durch Paarung von Hefewirtszellen mit Hefezellen, die die zielende DNS enthalten, eingeführt wird; oder
ii) der zielende DNS-Vektor ein lineares DNS-Fragment ist, wobei die zielende DNS-Sequenz durch die Insertion des selektierbaren Markergens zur Selektion der eukaryontischen Wirtszelle unterbrochen ist.

3. Ein homologes Rekombinationsverfahren zur Identifizierung und Isolierung eines Ziel-DNS-Fragments aus einer in eukaryontischen Wirtszellen konstruierten DNS-Fragmentbibliothek, wobei das Verfahren die Schritte umfaßt:
a) Bereitstellen einer DNS-Fragmentbibliothek in einer Population eukaryontischer Wirtszellen, in denen eine genetische Rekombination zwischen in die eukaryontischen Wirtszellen eingeführter DNS und in den eukaryontischen Wirtszellen vorhandener DNS durch homologe Rekombination stattfindet;
b) Einführen eines selektierbaren Markergens und einer zielenden DNS-Sequenz, die miteinander verbunden sind, in die Population der eukaryontischen Wirtszellen, die die DNS-Fragmentbibliothek enthalten, wodurch eine gemischte Population eukaryontischer Wirtszellen gebildet wird, wobei das selektierbare Markergen zur Selektion in der Wirtszelle ist, und die zielende DNS-Sequenz teilweise homolog zu einem Ziel-DNS-Fragment der eingeführten DNS ist,
c) Halten der gemischten Population eukaryontischer Wirtszellen unter geeigneten Bedingungen, um eine homologe Rekombination stattfinden zu lassen, wobei das Ziel-DNS-Fragment enthaltende eukaryontische Wirtszellen stabil mit dem selektierbaren Markergen und der in dem zielenden DNS-Vektor vorhandenen zielenden DNS-Sequenz transformiert sind als ein Ergebnis einer homologen Rekombination zwischen dem Ziel-DNS-Fragment und der zielenden DNS-Sequenz, und es werden stabil transformierte eukaryontische Wirtszellen mit einem selektierbaren Phänotyp gebildet; und
d) Kultivieren des Produkts von Schritt (c) unter geeigneten Bedingungen zur Selektion stabil transformierter eukaryontischer Wirtszellen mit einem selektierbaren Phänotyp.

4. Ein Verfahren gemäß Anspruch 3, worin (i) das zielende DNS-Molekül ein zielendes Plasmid mit beispielsweise einem in die zielende DNS-Sequenz eingeführten Doppelstrangbruch ist, oder
(ii) das Ziel-DNS-Fragment cDNS ist.

5. Ein Verfahren gemäß Anspruch 4, worin (A) das zielende Plasmid ein YIp Plasmid ist, und DNS-Fragmente in eukaryontischen Wirtszellen in künstlichen Chromosomen vorhanden sind, wobei die künstlichen Chromosomen zusätzlich sämtliche DNS-Sequenzen aufweisen, die für die Teilnahme der Chromosomen an der Wirtszellreplikation und Chromosomen-Segregation notwendig sind; oder (B)das Ziel-DNS-Fragment ist: eine Säuger DNS-Sequenz, die eine intergene DNS-Sequenz ist; eine menschliche DNS-Sequenz, die eine intergene DNS-Sequenz ist; eine pflanzliche DNS-Sequenz, die eine intergene DNS-Sequenz ist; ein Säuger-Gen oder ein Teil davon; ein menschliches Gen oder ein Teil davon; oder ein pflanzliches Gen oder ein Teil davon.

6. Ein Verfahren gemäß Anspruch 5, worin das selektierbare Markergen aus der Gruppe der Gene gewählt ist, die eukaryontischen Wirtszellen einen selektierbaren Phänotyp verleihen, und der selektierbare Phänotyp ist: Antibiotikumresistenz, nutrititive Prototrophie, Metallionentoleranz, Fähigkeit, den Zellzyklus zu durchlaufen oder Expression eines Zelloberflächenmarkers.

7. Ein homologes Rekombinationsverfahren zur Identifizierung und Isolierung eines Ziel-DNS-Fragments in einer in Hefezellen konstruierten DNS-Fragmentbibliothek, wobei das Verfahren die Schritte umfaßt:
a) Bereitstellen einer DNS-Fragmentbibliothek in einer Population Hefewirtszellen, worin DNS-Fragmente in künstlichen Hefechromosomen vorhanden sind, und eine Hefewirtszelle ein künstliches Hefechromosom oder eine low-copy-number künstlicher Hefechromosomen enthält;
b) Einführen eines selektierbaren Markergens zur Selektion in Hefe, das an eine zielende DNS gebunden ist, die teilweise homolog zu einem Ziel-DNS-Fragment ist, in die Hefezellen, die die DNS-Fragmentbibliothek enthalten, wodurch eine gemischte Population Hefezellen gebildet wird;
c) Halten der gemischten Hefezellpopulation unter geeigneten Bedingungen für eine homologe Rekombination zwischen zielender DNS und in den künstlichen Hefechromosomen vorhandenen Ziel-DNS-Fragmenten, wodurch Hefezellen, die das Ziel-DNS-Fragment enthalten, stabil mit dem selektierbaren Markergen und der zielenden DNS-Sequenz transformiert sind als ein Ergebnis der homologen Rekombination zwischen dem Ziel-DNS-Fragment und der zielenden DNS-Sequenz, und es werden stabil transformierte Hefezellen mit einem selektierbaren Phänotyp gebildet; und
d) Kultivieren des Produkts von Schritt (c) unter geeigneten Bedingungen zur Selektion von Hefezellen mit einem selektierbaren Phänotyp.

8. Ein Verfahren gemäß Anspruch 7, worin das zielende DNS-Molekül ein lineares DNS-Fragment ist, worin die zielende DNS-Sequenz durch die Insertion des selektierbaren Markers für Hefe in der zielenden DNS unterbrochen wird.

9. Ein homologes Rekombinationsverfahren zur Identifizierung und Isolierung eines Ziel-DNS-Fragments in einer in Hefezellen konstruierten DNS-Fragmentbibliothek, wobei das Verfahren die Schritte umfaßt:
a) Bereitstellen einer DNS-Fragmentbibliothek in einer Population aus Hefewirtszellen, worin DNS-Fragmente in künstlichen Hefechromosomen vorhanden sind, und eine Hefewirtszelle ein künstliches Hefechromosom oder eine low-copy-number künstlicher Hefechromosomen enthält;
b) Einführen eines in Hefe nicht-replizierenden zielenden DNS-Vektors in die Hefezellen, die die DNS-Fragmentbibliothek enthalten, wobei der zielende DNS-Vektor ein selektierbares Markergen zur Selektion in Hefe besitzt, und die zielende DNS teilweise zu einem Ziel-DNS-Fragment homolog ist, wodurch eine gemischte Hefezellpopulation gebildet wird;
c) Halten der gemischten Hefezellpopulation unter geeigneten Bedingungen für eine homologe Rekombination zwischen zielender DNS und in den künstlichen Hefechromosomen vorhandenen Ziel-DNS-Fragmenten, wodurch Hefezellen, die das Ziel-DNS-Fragment enthalten, stabil mit dem selektierbaren Markergen und der im zielenden DNS-Vektor enthaltenen zielenden DNS-Sequenz transformiert sind als ein Ergebnis der homologen Rekombination zwischen dem Ziel-DNS-Fragment und der zielenden DNS-Sequenz, und es werden stabil transformierte Hefezellen mit einem selektierbaren Phänotyp gebildet; und
d) Kultivieren des Produkts von Schritt (c) unter geeigneten Bedingungen zur Selektion von Hefezellen mit einem selektierbaren Phänotyp.

10. Ein Verfahren gemäß Anspruch 9, worin der zielende DNS-Vektor ein Plasmid ist und der selektierbare Phänotyp ist: Antibiotikumresistenz, nutrititive Prototrophie, Metallionentoleranz, Fähigkeit, den Zellzyklus zu durchlaufen oder Expression eines Zelloberflächenmarkers; und beispielsweise worin das zielende Plasmid einen in die zielende DNS eingeführten Doppelstrangbruch besitzt.

11. Ein homologes Rekombinationsverfahren zur Identifizierung und Isolierung eines Ziel-DNS-Fragments in einer künstlichen Hefechromosombibliothek, wobei das Verfahren die Schritte umfaßt:
a) Bereitstellen einer künstlichen Hefechromosombibliothek in einer Population Hefewirtszellen, worin DNS-Fragmente in künstlichen Hefechromosomen vorhanden sind und eine Einzelkopie oder eine low-copy-number künstlicher Hefechromosomen in Hefewirtszellen vorhanden ist;
b) Einführen eines zielenden Plasmids, das ein in Hefe nicht-replizierendes bakterielles Plasmid ist, in die künstliche Hefechromosombibliothek von (a), wobei das zielende Plasmid ein selektierbares Markergen zur Selektion in Hefe und eine zielende DNS mit einem Doppelstrangbruch besitzt;
c) Halten des Produkts von Schritt (b) unter geeigneten Bedingungen für eine homologe Rekombination zwischen der zielenden DNS und in den künstlichen Hefechromosomen vorhandenen Ziel-DNS-Fragmenten, wobei homologe Rekombination zwischen einem Ziel-DNS-Fragment und zielender DNS Hefewirtszellen bildet, die einen selektierbaren Phänotyp besitzen; und
d) Selektion der Hefewirtszellen, die den in Schritt (c) verliehenen selektierbaren Phänotyp besitzen.

12. Ein Verfahren gemäß Anspruch 10 oder Anspruch 11, worin das Ziel-DNS-Fragment ist: eine Säuger DNS-Sequenz, die eine intergene DNS-Sequenz ist; eine menschliche DNS-Sequenz, die eine intergene DNS-Sequenz ist; eine pflanzliche DNS-Sequenz, die eine intergene DNS-Sequenz ist; ein Säuger-Gen oder ein Teil davon; ein menschliches Gen oder ein Teil davon; oder ein pflanzliches Gen oder ein Teil davon.

13. Ein mit dem Verfahren gemäß Anspruch 6 oder Anspruch 12 isoliertes DNS-Ziel-Fragment, das eine Säuger DNS-Sequenz ist, die eine intergene DNS-Sequenz ist; eine menschliche DNS-Sequenz, die eine intergene DNS-Sequenz ist; eine pflanzliche DNS-Sequenz, die eine intergene DNS-Sequenz ist; ein Säuger-Gen oder ein Teil davon; ein menschliches Gen oder ein Teil davon; oder ein pflanzliches Gen oder ein Teil davon.

14. Ein Verfahren gemäß Anspruch 11, worin der selektierbare Phänotyp ist: Antibiotikumresistenz, nutrititive Prototrophie, Metallionentoleranz, Fähigkeit, den Zellzyklus zu durchlaufen oder Expression eines Zelloberflächenmarkers.

15. Ein Verfahren gemäß Anspruch 9, das des weiteren Isolieren angrenzender DNS-Segmente aus der DNS-Fragmentbibliothek umfaßt, wobei das Verfahren die Schritte umfaßt:
a) Subklonieren eines Ziel-DNS-Fragmentendes, das mit dem Verfahren gemäß Anspruch 9 erhalten wird, in einen zielenden DNS-Vektor, der in Hefe nicht-replizierend ist, wobei der zielende DNS-Vektor ein selektierbares Markergen zur Selektion in Hefe besitzt, wodurch ein zielender DNS-Vektor gebildet wird, der subklonierte DNS enthält, die das Ende des zuvor isolierten DNS-Fragments ist;
b) Einführen des zielenden Vektors, der in (a) als nachfolgende zielende DNS gebildet wird, in die bereitgestellte DNS-Fragmentbibliothek;
c) Halten des Produkts von (b) unter geeigneten Bedingungen für eine homologe Rekombination zwischen zielender DNS und Ziel-DNS-Fragmenten in der DNS-Fragmentbibliothek, wodurch Hefezellen gebildet werden, die stabil mit dem selektierbaren Markergen und zielender DNS transformiert sind als ein Ergebnis der homologen Rekombination zwischen dem Ziel-DNS-Fragment und der zielenden DNS-Sequenz und einen selektierbaren Phänotyp besitzen;
d) Kultivieren des Produkts von Schritt (c) unter geeigneten Bedingungen zur Selektion von Hefezellen, in denen eine homologe Rekombination stattgefunden hat;
e) Selektion der in Schritt (d) gebildeten Hefezellen, die einen selektierbaren Phänotyp besitzen;
f) Subklonieren eines Ziel-DNS-Fragmentendes, das in (e) selektierten Hefezellen vorhandenen ist, in einen zielenden DNS-Vektor wie in Schritt (a); und
g) Wiederholen der Schritte (b) bis (e) je nach Bedarf; und beispielsweise worin der zielende DNS-Vektor ein bakterielles Plasmid mit einem Doppelstrangbruch innerhalb der zielenden DNS ist, und der selektierbare Phänotyp ausgewählt ist aus der Gruppe enthaltend: Antibiotikumresistenz, nutrititive Prototrophie, Metallionentoleranz und Komplementation der unvollständigen Funktion eines Gens, das für das Durchlaufen des Zellzyklus essentiell ist.

16. Saccharomyces cerevisiae mit einer chromosomalen Deletion der gesamten codierenden Region zweier oder mehrerer selektierbarer Markergene, die beispielsweise nutrititive Prototrophie verleihen.

17. Ein
Saccharomyces cerevisiae TD7-16d (ATCC 74010);
Saccharomyces cerevisiae IV-16d; und
Saccharomyces cerevisiae MGD131-10c.

18. Ein Verfahren zur Herstellung eines Plasmids p184DLARG und funktioneller Äquivalente davon, einschließlich des Schrittes zur Bereitstellung des Plasmids mit Hefe-Gen ARG4 als ein selektierbarer Marker.

19. Ein Verfahren zur Herstellung eines in Hefe nicht replizierenden Plasmids, das die Schritte umfaßt:
a) Bereitstellen eines selektierbaren Hefe-Markergens;
b) Bereitstellen eines bakteriellen Replikationsursprungs;
c) Bereitstellen eines selektierbaren Bakterien-Markergens; und
d) Bereitstellen einer Klonierungsstelle zur Insertion einer zielenden DNS.

20. Saccharomyces cerevisiae mit einer chromosomalen Deletion des Argininsuccinat-Lyase-Gens und einer chromosomalen Deletion des N-(5'-phosphoribosyl)-anthranilat-Isomerase-Gens.

## Revendications (Revendications pour l'(les) Etat(s) contractant(s) suivant(s): AT, BE, CH, LI, DE, DK, FR, GB, GR, IT, LU, NL, SE)

1. Procédé de recombinaison homologue pour identifier et isoler un fragment d'ADN cible à partir d'une banque de fragments d'ADN construite dans des cellules hôtes eucaryotes, comprenant les étapes consistant à:
a) disposer de la banque de fragments d'ADN introduits dans une population de cellules hôtes eucaryotes dans laquelle il se produit une recombinaison génétique entre l'ADN introduit dans les cellules hôtes eucaryotes et l'ADN présent dans les cellules hôtes eucaryotes par recombinaison homologue;
b) incorporer un gène marqueur sélectionnable et une séquence d'ADN de criblage liés l'un à l'autre, dans la population de cellules hôtes eucaryotes contenant la banque de fragments d'ADN de façon à obtenir une population mixte de cellules hôtes eucaryotes, le gène marqueur sélectionnable étant destiné à la sélection des cellules hôtes et la séquence d'ADN de criblage étant partiellement homologue à un fragment d'ADN cible de l'ADN introduit;
c) maintenir la population mixte de cellules hôtes eucaryotes dans des conditions appropriées pour qu'il se produise une recombinaison homologue, de façon à ce que seules les cellules hôtes eucaryotes contenant le fragment d'ADN cible de l'ADN introduit soient transformées de manière stable avec le gène marqueur sélectionnable et la séquence d'ADN de criblage à la suite d'une recombinaison homologue entre le fragment d'ADN cible et la séquence d'ADN de criblage et que des cellules hôtes eucaryotes transformées de manière stable présentant un phénotype sélectionnable soient produites; et
d) cultiver le produit de l'étape (c) dans des conditions appropriées pour la sélection de cellules hôtes eucaryotes transformées de manière stable présentant un phénotype sélectionnable.

2. Procédé selon la revendication 1 dans lequel
(i) les cellules hôtes eucaryotes sont des cellules hôtes de levure, l'ADN de criblage est présent dans un vecteur linéaire capable de se répliquer qui se trouve dans des levures de signe de conjugaison opposé à celui des levures hôtes et sont appelées levures contenant l'ADN de criblage et l'ADN de criblage est introduit dans des levures hôtes en croisant des levures hôtes avec des levures contenant l'ADN de criblage; ou
(ii) le vecteur d'ADN de criblage est un fragment d'ADN linéaire dans lequel la séquence d'ADN de criblage est interrompue par l'insertion du gène marqueur sélectionnable destiné à sélectionner les cellules eucaryotes hôtes.

3. Procédé de recombinaison homologue pour identifier et isoler un fragment d'ADN cible à partir d'une banque de fragments d'ADN construite dans des cellules hôtes eucaryotes, comprenant les étapes consistant à:
a) disposer d'une banque de fragments d'ADN dans une population de cellules hôtes eucaryotes dans laquelle il se produit une recombinaison génétique entre l'ADN introduit dans les cellules hôtes eucaryotes et l'ADN présent dans les cellules hôtes eucaryotes par recombinaison homologue;
b) introduire un gène marqueur sélectionnable et une séquence d'ADN de criblage liés l'un à l'autre, dans une population de cellules hôtes eucaryotes contenant la banque de fragments d'ADN de façon à obtenir une population mixte de cellules hôtes eucaryotes, le gène marqueur sélectionnable étant destiné à sélectionner les cellules hôtes et la séquence d'ADN de criblage étant partiellement homologue à un fragment d'ADN cible de l'ADN introduit;
c) maintenir la population mixte de cellules hôtes eucaryotes dans des conditions appropriées pour qu'il se produise une recombinaison homologue, de façon à ce que les cellules hôtes eucaryotes contenant le fragment d'ADN cible soient transformées de manière stable avec le gène marqueur sélectionnable et la séquence d'ADN de criblage présente dans le vecteur d'ADN de criblage à la suite d'une recombinaison homologue entre le fragment d'ADN cible et la séquence d'ADN de criblage et que des cellules hôtes eucaryotes transformées de manière stable présentant un phénotype sélectionnable soient produites; et
d) cultiver le produit de l'étape (c) dans des conditions appropriées pour la sélection de cellules hôtes eucaryotes transformées de manière stable présentant un phénotype sélectionnable.

4. Procédé selon la revendication 3 dans lequel
(i) la molécule d'ADN de criblage est un plasmide de criblage, possédant par exemple une coupure des deux brins introduite au sein de la séquence d'ADN de criblage, ou
(ii) le fragment d'ADN cible est un ADNc.

5. Procédé selon la revendication 4 dans lequel
(A) le plasmide de criblage est un plasmide YIp et les fragments d'ADN se trouvent dans des cellules hôtes eucaryotes dans des chromosomes artificiels, les chromosomes artificiels contenant en outre toutes les séquences d'ADN nécessaires pour que le chromosome participe à la réplication et à la ségrégation des chromosomes de la cellule hôte; ou
(B) le fragment d'ADN cible est: une séquence d'ADN mammalienne qui est une séquence d'ADN intergénique; une séquence d'ADN humain qui est une séquence d'ADN intergénique; une séquence d'ADN végétal qui est une séquence d'ADN intergénique; un gène ou un fragment de gène mammalien; un gène ou un fragment de gène humain; un gène ou un fragment de gène végétal.

6. Procédé selon la revendication 5 dans lequel le gène marqueur sélectionnable est choisi au sein du groupe constitué par les gènes qui confèrent un phénotype sélectionnable aux cellules hôtes eucaryotes et le phénotype sélectionnable est: une résistance à un antibiotique, une prototrophie pour un élément nutritif, une tolérance à un ion métallique, l'aptitude à poursuivre le cycle cellulaire ou l'expression d'un marqueur membranaire.

7. Procédé de recombinaison homologue pour identifier et isoler un fragment d'ADN cible dans une banque de fragments d'ADN construite dans des cellules de levure, comprenant les étapes consistant à:
a) disposer d'une banque de fragments d'ADN dans une population de cellules-hôtes de levure dans laquelle les fragments d'ADN se trouvent dans des chromosomes de levure artificiels et une cellule hôte de levure contient un chromosome de levure artificiel unique ou un petit nombre de chromosomes de levure artificiels;
b) introduire dans les cellules de levure contenant la banque de fragments d'ADN un gène marqueur sélectionnable destiné à sélectionner les levures lié à un fragment d'ADN de criblage partiellement homologue à un fragment d'ADN cible de façon à obtenir une population mixte de cellules de levures;
c) maintenir la population mixte de cellules de levure dans des conditions appropriées pour qu'il se produise une recombinaison homologue entre les fragments d'ADN de criblage et les fragments d'ADN cible présents dans les chromosomes de levure artificiels, de façon à ce que les cellules de levure contenant le fragment d'ADN cible soient transformées de manière stable avec le gène marqueur sélectionnable et la séquence d'ADN de criblage à la suite d'une recombinaison homologue entre le fragment d'ADN cible et la séquence d'ADN de criblage et que des cellules de levure transformées de manière stable présentant un phénotype sélectionnable soient produites; et
d) cultiver le produit de l'étape (c) dans des conditions appropriées pour la sélection de cellules de levure transformées de manière stable présentant un phénotype sélectionnable.

8. Procédé selon la revendication 7 dans lequel la molécule d'ADN de criblage est un fragment d'ADN linéaire où la séquence d'ADN de criblage est interrompue par l'insertion du marqueur sélectionnable pour la levure.

9. Procédé de recombinaison homologue pour identifier et isoler un fragment d'ADN cible dans une banque de fragments d'ADN construite dans des cellules de levure, comprenant les étapes consistant à:
a) disposer d'une banque de fragments d'ADN dans une population de cellules-hôtes de levure dans laquelle les fragments d'ADN se trouvent dans des chromosomes de levure artificiels, une cellule-hôte de levure contenant un chromosome de levure artificiel unique ou un petit nombre de chromosomes de levure artificiels;
b) introduire dans les cellules de levure contenant la banque de fragments d'ADN un vecteur d'ADN de criblage qui ne se réplique pas dans la levure, le vecteur d'ADN de criblage possédant un gène marqueur sélectionnable destiné à la sélection de levures et un fragment d'ADN partiellement homologue à un fragment d'ADN cible de façon à obtenir une population mixte de cellules de levures;
c) maintenir la population mixte de cellules de levure dans des conditions appropriées pour qu'il se produise une recombinaison homologue entre les fragments d'ADN de criblage et les fragments d'ADN cible présents dans les chromosomes de levure artificiels, de façon à ce que les cellules de levure contenant le fragment d'ADN cible soient transformées de manière stable avec le gène marqueur sélectionnable et la séquence d'ADN de criblage à la suite d'une recombinaison homologue entre le fragment d'ADN cible et la séquence d'ADN de criblage et que des cellules de levure transformées de manière stable présentant un phénotype sélectionnable soient produites; et
d) cultiver le produit de l'étape (c) dans des conditions appropriées pour la sélection de cellules de levure transformées de manière stable présentant un phénotype sélectionnable.

10. Procédé selon la revendication 9 dans lequel le vecteur d'ADN de criblage est un plasmide et le phénotype sélectionnable est : une résistance aux antibiotiques, une prototrophie pour un élément nutritif, une tolérance à un ion métallique, l'aptitude à poursuivre le cycle cellulaire, ou l'expression d'un marqueur membranaire; et par exemple dans lequel le plasmide de criblage possède une coupure des deux brins introduite au sein de l'ADN de criblage.

11. Procédé de recombinaison homologue pour identifier et isoler un fragment d'ADN cible dans une banque de chromosomes artificiels de levure, comprenant les étapes consistant à:
a) disposer d'une banque de chromosomes artificiels de levure dans une population de cellules-hôtes de levure dans laquelle les fragments d'ADN se trouvent dans des chromosomes de levure artificiels, les cellules-hôte de levure contenant un chromosome de levure artificiel unique ou un petit nombre de chromosomes de levure artificiels;
b) introduire dans la banque de chromosomes artificiels de levure de l'étape (a) un plasmide de criblage qui est un plasmide bactérien qui ne se réplique pas dans la levure, le plasmide de criblage possédant un gène marqueur sélectionnable destiné à la sélection dans la levure et un fragment d'ADN de criblage contenant une coupure des deux brins;
c) maintenir le produit de l'étape (b) dans des conditions appropriées pour une recombinaison homologue entre l'ADN de criblage et les fragments d'ADN cible présents dans les chromosomes de levure artificiels, de façon à ce qu'une recombinaison homologue entre un fragment d'ADN cible et l'ADN de criblage produise des cellules hôtes de levure présentant un phénotype sélectionnable; et
d) sélectionner les cellules-hôtes de levure possédant le phénotype sélectionnable conféré à l'étape (c).

12. Procédé selon la revendication 10 ou 11, dans lequel le fragment d'ADN cible est: une séquence d'ADN mammalienne qui est une séquence d'ADN intergénique; une séquence d'ADN humain qui est une séquence d'ADN intergénique; une séquence d'ADN végétal qui est une séquence d'ADN intergénique; un gène ou un fragment de gène mammalien; un gène ou un fragment de gène humain; un gène ou un fragment de gène végétal.

13. Fragment d'ADN cible isolé à l'aide du procédé selon la revendication 6 ou 12, qui est: une séquence d'ADN mammalienne qui est une séquence d'ADN intergénique; une séquence d'ADN humain qui est une séquence d'ADN intergénique; une séquence d'ADN végétal qui est une séquence d'ADN intergénique; un gène ou un fragment de gène mammalien; un gène ou un fragment de gène humain; un gène ou un fragment de gène végétal.

14. Procédé selon la revendication 11 dans lequel phénotype sélectionnable est: une résistance à un antibiotique, une prototrophie pour un élément nutritif, une tolérance à un ion métallique, l'aptitude à poursuivre le cycle cellulaire ou l'expression d'un marqueur membranaire.

15. Procédé selon la revendication 9 comprenant l'isolement de segments d'ADN adjacents à partir de la banque de fragments d'ADN, comprenant les étapes consistant à:
a) souscloner une extrémité d'un fragment d'ADN obtenu par le procédé de la revendication 9 dans un vecteur d'ADN de criblage qui ne se réplique pas dans la levure, le vecteur d'ADN de criblage possédant un gène marqueur sélectionnable destiné à sélectionner les levures, de façon à produire un vecteur d'ADN de criblage contenant l'ADN souscloné correspondant à l'extrémité du fragment d'ADN cible isolé auparavant;
b) introduire dans la banque de fragments d'ADN le vecteur de criblage produit en (a) en vue du criblage ultérieur;
c) maintenir le produit de l'étape (b) dans des conditions appropriées pour une recombinaison homologue entre les fragments d'ADN cible et les fragments d'ADN de criblage dans la banque de fragments d'ADN, de façon à produire des cellules de levure transformées de manière stable avec le gène marqueur sélectionnable et l'ADN de criblage à la suite d'une recombinaison homologue entre le fragment d'ADN cible et la séquence d'ADN de criblage et possédant un phénotype sélectionnable;
d) cultiver le produit de l'étape (c) dans des conditions appropriées pour la sélection de cellules de levure dans lesquelles une recombinaison homologue a eu lieu;
e) sélectionner les cellules de levure produites à l'étape (d), présentant un phénotype sélectionnable;
f) sous-cloner une extrémité du fragment d'ADN cible présent dans des cellules de levure sélectionnées au point (e) dans un vecteur d'ADN de criblage comme à l'étape (a); et
g) répéter les étapes (b) à (e) si besoin est; et par exemple, où le vecteur d'ADN de criblage est un plasmide bactérien possédant une coupure sur deux brins dans l'ADN de criblage et le phénotype sélectionnable est choisi au sein du groupe constitué de: une résistance à un antibiotique, une prototrophie pour un élément nutritif, la tolérance à un ion métallique et le complément du dysfonctionnement d'un gène essentiel pour la poursuite du cycle cellulaire.

16. Souche de Saccharomyces cerevisiae portant une délétion chromosomique de la totalité de la région codante de deux ou plusieurs marqueurs sélectionnables qui confèrent par exemple une prototrophie pour un élément nutritif.

17. Souche de Saccharomyces cerevisiae choisie parmi les souches suivantes:
Saccharomyces cerevisiae TD7-16d (ATCC 74010);
Saccharomyces cerevisiae IV-16d ; et
Saccharomyces cerevisiae MGD131-10c.

18. Plasmide p184DLARG et ses équivalents fonctionnels.

19. Plasmide incapable de se répliquer dans la levure, comprenant:
a) un gène marqueur sélectionnable de la levure;
b) une origine de réplication bactérienne;
c) un gène marqueur bactérien sélectionnable; et
d) un site de clonage pour l'insertion de l'ADN de criblage

20. Souche de Saccharomyces cerevisiae portant une délétion chromosomique du gène de l'argininosuccinate lyase et une délétion chromosomique du gène de la N-(5'-phosphoribosyl)-anthranilate isomérase.

## Revendications (Revendications pour l'(les) Etat(s) contractant(s) suivant(s): ES)

1. Procédé de recombinaison homologue pour identifier et isoler un fragment d'ADN cible à partir d'une banque de fragments d'ADN construite dans des cellules hôtes eucaryotes, comprenant les étapes consistant à:
a) disposer de la banque de fragments d'ADN introduits dans une population de cellules hôtes eucaryotes dans laquelle il se produit une recombinaison génétique entre l'ADN introduit dans les cellules hôtes eucaryotes et l'ADN présent dans les cellules hôtes eucaryotes par recombinaison homologue;
b) incorporer un gène marqueur sélectionnable et une séquence d'ADN de criblage liés l'un à l'autre, dans la population de cellules hôtes eucaryotes contenant la banque de fragments d'ADN de façon à obtenir une population mixte de cellules hôtes eucaryotes, le gène marqueur sélectionnable étant destiné à la sélection des cellules hôtes et la séquence d'ADN de criblage étant partiellement homologue à un fragment d'ADN cible de l'ADN introduit;
c) maintenir la population mixte de cellules hôtes eucaryotes dans des conditions appropriées pour qu'il se produise une recombinaison homologue, de façon à ce que seules les cellules hôtes eucaryotes contenant le fragment d'ADN cible de l'ADN introduit soient transformées de manière stable avec le gène marqueur sélectionnable et la séquence d'ADN de criblage à la suite d'une recombinaison homologue entre le fragment d'ADN cible et la séquence d'ADN de criblage et que des cellules hôtes eucaryotes transformées de manière stable présentant un phénotype sélectionnable soient produites; et
d) cultiver le produit de l'étape (c) dans des conditions appropriées pour la sélection de cellules hôtes eucaryotes transformées de manière stable présentant un phénotype sélectionnable.

2. Procédé selon la revendication 1 dans lequel
(i) les cellules hôtes eucaryotes sont des cellules hôtes de levure, l'ADN de criblage est présent dans un vecteur linéaire capable de se répliquer qui se trouve dans des levures de signe de conjugaison opposé à celui des levures hôtes et sont appelées levures contenant l'ADN de criblage et l'ADN de criblage est introduit dans des levures hôtes en croisant des levures hôtes avec des levures contenant l'ADN de criblage; ou
(ii) le vecteur d'ADN de criblage est un fragment d'ADN linéaire dans lequel la séquence d'ADN de criblage est interrompue par l'insertion du gène marqueur sélectionnable destiné à sélectionner les cellules eucaryotes hôtes.

3. Procédé de recombinaison homologue pour identifier et isoler un fragment d'ADN cible à partir d'une banque de fragments d'ADN construite dans des cellules hôtes eucaryotes, comprenant les étapes consistant à:
a) disposer d'une banque de fragments d'ADN dans une population de cellules hôtes eucaryotes dans laquelle il se produit une recombinaison génétique entre l'ADN introduit dans les cellules hôtes eucaryotes et l'ADN présent dans les cellules hôtes eucaryotes par recombinaison homologue;
b) introduire un gène marqueur sélectionnable et une séquence d'ADN de criblage liés l'un à l'autre, dans une population de cellules hôtes eucaryotes contenant la banque de fragments d'ADN de façon à obtenir une population mixte de cellules hôtes eucaryotes, le gène marqueur sélectionnable étant destiné à sélectionner les cellules hôtes et la séquence d'ADN de criblage étant partiellement homologue à un fragment d'ADN cible de l'ADN introduit;
c) maintenir la population mixte de cellules hôtes eucaryotes dans des conditions appropriées pour qu'il se produise une recombinaison homologue, de façon à ce que les cellules hôtes eucaryotes contenant le fragment d'ADN cible soient transformées de manière stable avec le gène marqueur sélectionnable et la séquence d'ADN de criblage présente dans le vecteur d'ADN de criblage à la suite d'une recombinaison homologue entre le fragment d'ADN cible et la séquence d'ADN de criblage et que des cellules hôtes eucaryotes transformées de manière stable présentant un phénotype sélectionnable soient produites; et
d) cultiver le produit de l'étape (c) dans des conditions appropriées pour la sélection de cellules hôtes eucaryotes transformées de manière stable présentant un phénotype sélectionnable.

4. Procédé selon la revendication 3 dans lequel
(i) la molécule d'ADN de criblage est un plasmide de criblage, possédant par exemple une coupure des deux brins introduite au sein de la séquence d'ADN de criblage, ou
(ii) le fragment d'ADN cible est un ADNc.

5. Procédé selon la revendication 4 dans lequel
(A) le plasmide de criblage est un plasmide YIp et les fragments d'ADN se trouvent dans des cellules hôtes eucaryotes dans des chromosomes artificiels, les chromosomes artificiels contenant en outre toutes les séquences d'ADN nécessaires pour que le chromosome participe à la réplication et à la ségrégation des chromosomes de la cellule hôte; ou
(B) le fragment d'ADN cible est: une séquence d'ADN mammalienne qui est une séquence d'ADN intergénique; une séquence d'ADN humain qui est une séquence d'ADN intergénique; une séquence d'ADN végétal qui est une séquence d'ADN intergénique; un gène ou un fragment de gène mammalien; un gène ou un fragment de gène humain; un gène ou un fragment de gène végétal.

6. Procédé selon la revendication 5 dans lequel le gène marqueur sélectionnable est choisi au sein du groupe constitué par les gènes qui confèrent un phénotype sélectionnable aux cellules hôtes eucaryotes et le phénotype sélectionnable est: une résistance à un antibiotique, une protptrophie pour un élément nutritif, une tolérance à un ion métallique, l'aptitude à poursuivre le cycle cellulaire ou l'expression d'un marqueur membranaire.

7. Procédé de recombinaison homologue pour identifier et isoler un fragment d'ADN cible dans une banque de fragments d'ADN construite dans des cellules de levure, comprenant les étapes consistant à:
a) disposer d'une banque de fragments d'ADN dans une population de cellules-hôtes de levure dans laquelle les fragments d'ADN se trouvent dans des chromosomes de levure artificiels et une cellule hôte de levure contient un chromosome de levure artificiel unique ou un petit nombre de chromosomes de levure artificiels;
b) introduire dans les cellules de levure contenant la banque de fragments d'ADN un gène marqueur sélectionnable destiné à sélectionner les levures lié à un fragment d'ADN de criblage partiellement homologue à un fragment d'ADN cible de façon à obtenir une population mixte de cellules de levures;
c) maintenir la population mixte de cellules de levure dans des conditions appropriées pour qu'il se produise une recombinaison homologue entre les fragments d'ADN de criblage et les fragments d'ADN cible présents dans les chromosomes de levure artificiels, de façon à ce que les cellules de levure contenant le fragment d'ADN cible soient transformées de manière stable avec le gène marqueur sélectionnable et la séquence d'ADN de criblage à la suite d'une recombinaison homologue entre le fragment d'ADN cible et la séquence d'ADN de criblage et que des cellules de levure transformées de manière stable présentant un phénotype sélectionnable soient produites; et
d) cultiver le produit de l'étape (c) dans des conditions appropriées pour la sélection de cellules de levure transformées de manière stable présentant un phénotype sélectionnable.

8. Procédé selon la revendication 7 dans lequel la molécule d'ADN de criblage est un fragment d'ADN linéaire où la séquence d'ADN de criblage est interrompue par l'insertion du marqueur sélectionnable pour la levure.

9. Procédé de recombinaison homologue pour identifier et isoler un fragment d'ADN cible dans une banque de fragments d'ADN construite dans des cellules de levure, comprenant les étapes consistant à:
a) disposer d'une banque de fragments d'ADN dans une population de cellules-hôtes de levure dans laquelle les fragments d'ADN se trouvent dans des chromosomes de levure artificiels, une cellule-hôte de levure contenant un chromosome de levure artificiel unique ou un petit nombre de chromosomes de levure artificiels;
b) introduire dans les cellules de levure contenant la banque de fragments d'ADN un vecteur d'ADN de criblage qui ne se réplique pas dans la levure, le vecteur d'ADN de criblage possédant un gène marqueur sélectionnable destiné à la sélection de levures et un fragment d'ADN partiellement homologue à un fragment d'ADN cible de façon à obtenir une population mixte de cellules de levures;
c) maintenir la population mixte de cellules de levure dans des conditions appropriées pour qu'il se produise une recombinaison homologue entre les fragments d'ADN de criblage et les fragments d'ADN cible présents dans les chromosomes de levure artificiels, de façon à ce que les cellules de levure contenant le fragment d'ADN cible soient transformées de manière stable avec le gène marqueur sélectionnable et la séquence d'ADN de criblage à la suite d'une recombinaison homologue entre le fragment d'ADN cible et la séquence d'ADN de criblage et que des cellules de levure transformées de manière stable présentant un phénotype sélectionnable soient produites; et
d) cultiver le produit de l'étape (c) dans des conditions appropriées pour la sélection de cellules de levure transformées de manière stable présentant un phénotype sélectionnable.

10. Procédé selon la revendication 9 dans lequel le vecteur d'ADN de criblage est un plasmide et le phénotype sélectionnable est : une résistance aux antibiotiques, une prototrophie pour un élément nutritif, une tolérance à un ion métallique, l'aptitude à poursuivre le cycle cellulaire, ou l'expression d'un marqueur membranaire; et par exemple dans lequel le plasmide de criblage possède une coupure des deux brins introduite au sein de l'ADN de criblage.

11. Procédé de recombinaison homologue pour identifier et isoler un fragment d'ADN cible dans une banque de chromosomes artificiels de levure, comprenant les étapes consistant à:
a) disposer d'une banque de chromosomes artificiels de levure dans une population de cellules-hôtes de levure dans laquelle les fragments d'ADN se trouvent dans des chromosomes de levure artificiels, une cellule-hôte de levure contenant un chromosome de levure artificiel unique ou un petit nombre de chromosomes de levure artificiels;
b) introduire dans la banque de chromosomes artificiels de levure de l'étape (a) un plasmide de criblage qui est un plasmide bactérien qui ne se réplique pas dans la levure, le plasmide de criblage possédant un gène marqueur sélectionnable destiné à la sélection dans la levure et un fragment d'ADN de criblage contenant une coupure des deux brins;
c) maintenir le produit de l'étape (b) dans des conditions appropriées pour une recombinaison homologue entre l'ADN de criblage et les fragments d'ADN cible présents dans les chromosomes de levure artificiels, de façon à ce qu'une recombinaison homologue entre un fragment d'ADN cible et l'ADN de criblage produise des cellules hôtes de levure présentant un phénotype sélectionnable; et
d) sélectionner les cellules-hôtes de levure possédant le phénotype sélectionnable conféré à l'étape (c).

12. Procédé selon la revendication 10 ou 11, dans lequel le fragment d'ADN cible est: une séquence d'ADN mammalienne qui est une séquence d'ADN intergénique; une séquence d'ADN humain qui est une séquence d'ADN intergénique; une séquence d'ADN végétal qui est une séquence d'ADN intergénique; un gène ou un fragment de gène mammalien; un gène ou un fragment de gène humain; un gène ou un fragment de gène végétal.

13. Fragment d'ADN cible isolé à l'aide du procédé selon la revendication 6 ou 12, qui est: une séquence d'ADN mammalienne qui est une séquence d'ADN intergénique; une séquence d'ADN humain qui est une séquence d'ADN intergénique; une séquence d'ADN végétal qui est une séquence d'ADN intergénique; un gène ou un fragment de gène mammalien; un gène ou un fragment de gène humain; un gène ou un fragment de gène végétal.

14. Procédé selon la revendication 11 dans lequel phénotype sélectionnable est: une résistance à un antibiotique, une prototrophie pour un élément nutritif, une tolérance à un ion métallique, l'aptitude à poursuivre le cycle cellulaire ou l'expression d'un marqueur membranaire.

15. Procédé selon la revendication 9 comprenant l'isolement de segments d'ADN adjacents à partir de la banque de fragments d'ADN, comprenant les étapes consistant à:
a) souscloner une extrémité d'un fragment d'ADN obtenu par le procédé de la revendication 9 dans un vecteur d'ADN de criblage qui ne se réplique pas dans la levure, le vecteur d'ADN de criblage possédant un gène marqueur sélectionnable destiné à sélectionner les levures, de façon à produire un vecteur d'ADN de criblage contenant l'ADN souscloné correspondant à l'extrémité du fragment d'ADN cible isolé auparavant;
b) introduire dans la banque de fragments d'ADN le vecteur de criblage produit en (a) en vue du criblage ultérieur;
c) maintenir le produit de l'étape (b) dans des conditions appropriées pour une recombinaison homologue entre les fragments d'ADN cible et les fragments d'ADN de criblage dans la banque de fragments d'ADN, de façon à produire des cellules de levure transformées de manière stable avec le gène marqueur sélectionnable et l'ADN de criblage à la suite d'une recombinaison homologue entre le fragment d'ADN cible et la séquence d'ADN de criblage et possédant un phénotype sélectionnable;
d) cultiver le produit de l'étape (c) dans des conditions appropriées pour la sélection de cellules de levure dans lesquelles une recombinaison homologue a eu lieu;
e) sélectionner les cellules de levure produites à l'étape (d), présentant un phénotype sélectionnable;
f) sous-cloner une extrémité du fragment d'ADN cible présent dans des cellules de levure sélectionnées au point (e) dans un vecteur d'ADN de criblage comme à l'étape (a); et
g) répéter les étapes (b) à (e) si besoin est; et par exemple, où le vecteur d'ADN de criblage est un plasmide bactérien possédant une coupure sur deux brins dans l'ADN de criblage et le phénotype sélectionnable est choisi au sein du groupe constitué de: une résistance à un antibiotique, une prototrophie pour un élément nutritif, la tolérance à un ion métallique et le complément du dysfonctionnement d'un gène essentiel pour la poursuite du cycle cellulaire.

16. Souche de Saccharomyces cerevisiae portant une délétion chromosomique de la totalité de la région codante de deux ou plusieurs marqueurs sélectionnables qui confèrent par exemple une prototrophie pour un élément nutritif.

17. Souche de Saccharomyces cerevisiae choisie parmi les souches suivantes:
Saccharomyces cerevisiae TD7-16d (ATCC 74010);
Saccharomyces cerevisiae IV-16d ; et
Saccharomyces cerevisiae MGD131-10c.

18. Procédé de fabrication d'un plasmide p184DLARG et ses équivalents fonctionnels comprenant l'étape consistant à munir le plasmide du gène de levure ARG4 en tant que marqueur sélectionnable.

19. Procédé de fabrication d'un plasmide incapable de se répliquer dans la levure, comprenant les étapes consistant à:
a) fournir un gène marqueur sélectionnable de la levure;
b) fournir une origine de réplication bactérienne;
c) fournir un gène marqueur bactérien sélectionnable; et
d) fournir un site de clonage pour l'insertion de l'ADN de criblage

20. Souche de Saccharomyces cerevisiae portant une délétion chromosomique du gène de l'argininosuccinate lyase et une délétion chromosomique du gène de la N-(5'-phosphoribosyl)-anthranilate isomérase.
